(19) Europäisches Patentamt
European Patent Office
Office européen des brevets

(11) **EP 4 021 425 B1**

(12) # EUROPEAN PATENT SPECIFICATION

(45) Date of publication and mention
of the grant of the patent:
**16.04.2025 Bulletin 2025/16**

(21) Application number: **20861492.5**

(22) Date of filing: **02.09.2020**

(51) International Patent Classification (IPC):
*A61K 9/51* (2006.01)     *C12N 15/88* (2006.01)
*A61K 48/00* (2006.01)     *A61K 9/107* (2006.01)

(52) Cooperative Patent Classification (CPC):
**A61K 9/5138; A61K 9/1075; A61K 9/5146;
A61K 9/5153; A61K 9/5192; A61K 31/7105;
A61K 31/711; A61K 38/26; A61K 47/34;
C12N 15/88;** A61K 9/5161

(86) International application number:
**PCT/US2020/048986**

(87) International publication number:
**WO 2021/046078 (11.03.2021 Gazette 2021/10)**

(54) **COMB POLYMER AND BLOCK COPOLYMER STABILIZED NANOPARTICLES ENCAPSULATING NUCLEIC ACIDS AND OTHER SOLUBLE HYDROPHILIC COMPOUNDS**

KAMMPOLYMER- UND BLOCKCOPOLYMERSTABILISIERTE NANOTEILCHEN ZUR VERKAPSELUNG VON NUKLEINSÄUREN UND ANDEREN LÖSLICHEN HYDROPHILEN VERBINDUNGEN

NANOPARTICULES STABILISÉES PAR UN COPOLYMÈRE SÉQUENCÉ ET UN POLYMÈRE EN PEIGNE ENCAPSULANT DES ACIDES NUCLÉIQUES ET D'AUTRES COMPOSÉS HYDROPHILES SOLUBLES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **04.09.2019 US 201962895695 P**

(43) Date of publication of application:
**06.07.2022 Bulletin 2022/27**

(73) Proprietor: **The Trustees of Princeton University Princeton, NJ 08544 (US)**

(72) Inventors:
• **PAGELS, Robert, F.**
**Princeton, NJ 08542 (US)**
• **MARKWALTER, Chester, E.**
**Princeton, NJ 08542 (US)**
• **PRUD'HOMME, Robert, K.**
**Lawrenceville, NJ 08648 (US)**

(74) Representative: **FRKelly
Waterways House
Grand Canal Quay
Dublin D02 PD39 (IE)**

(56) References cited:
**WO-A1-2013/188979     US-A1- 2004 023 393
US-A1- 2017 209 386     US-A1- 2017 209 386
US-A1- 2018 009 924     US-A1- 2019 008 788
US-A1- 2019 008 788**

• **PALLAVI THAPLYAL, PHILIP C.BEVILACQUA: "Experimental Approaches for Measuring pKa's in RNA and DNA", METHODS IN ENZYMOLOGY, vol. 549, 25 November 2014 (2014-11-25), pages 189 - 219, XP055909617**

Description

CROSS-REFERENCE TO RELATED APPLICATIONS

This application claims priority to U.S. Provisional Patent Application No. 62/895,695, filed September 4, 2019.

FIELD OF THE INVENTION

The present invention relates to the process of forming nanoparticles having a hydrophilic core which are stabilized by comb polymers, the subsequent processing of those nanoparticles, and the compositions thereof.

BACKGROUND OF THE INVENTION

Biologics, including proteins, peptides, ribonucleic acid (RNA), deoxyribonucleic acid (DNA), and polysaccharides, are an important class of pharmaceuticals due to their high potency and specificity. However, they are limited in how they can be delivered to patients. Most biologics are not orally bioavailable and require parenteral administration. Some biologics, including peptides and nucleic acids, have short half-lives and are rapidly degraded by proteases and RNases or DNases respectively and rapidly cleared. Nucleic acids and other biologics can also be recognized and cleared by the immune system. An additional limitation of biologics is that their high water solubility prevents them from permeating cell membranes. Therefore, most biologic therapeutics have been limited to targeting extracellular receptors. For these reasons, delivery vehicles including nanocarriers and microcarriers that protect the biologic form degradation, prolong half-lives, reduce the frequency of injection, and/or allow for intracellular delivery are required.

World Patents WO 2017/112828 A1 and WO 2015/200054, presented methods for encapsulating hydrophilic compounds including biologics in nanoparticles and microparticles. This invention was termed "inverse Flash NanoPrecipitation" (iFNP). In the iFNP process, a hydrophilic active agent such as a biologic and stabilizing block copolymer are dissolved in a polar solvent stream which is rapidly mixed with a non-polar non-solvent stream. Upon mixing, the hydrophilic active precipitates, forming the nanoparticle core. The hydrophilic block(s) of the stabilizing polymer stick to the nanoparticle surface, and the hydrophobic block(s) of the stabilizing polymer face the external aqueous phase.

Until now, the encapsulation of nucleic acids including RNA by iFNP has not been demonstrated. Here we show that iFNP can be used to encapsulate RNA with the same well-defined block copolymer stabilizers that have previously been described. This was unexpected because the block copolymers commonly used in the iFNP process have include an anionic (negatively charged) section which was expected to have unfavorable interactions with negatively charged nucleic acids. We also introduce a new class of stabilizers that have never been used before in iFNP, comb polymers, and show that they can be used to encapsulate RNA and other biologics.

The prior art cited in the Supplementary European search report, namely, US 2019/008788 A1, US 2017/209386 A1 and WO 2013/188979 A1, may be useful for understanding the background to the present invention.

SUMMARY OF THE INVENTION

The present invention is defined by the appended independent claims. Optional features are defined by the dependent claims. In an example, we describe nanoparticles comprising of a hydrophilic core that are stabilized by block copolymers or comb polymers. These nanoparticles can range in size from about 10 nm to about 5000 nm. In an embodiment of this invention, the hydrophilic core can include water-soluble small molecules, proteins, peptides, nucleic acids including DNA and RNA, and/or polysaccharides (also referred as a hydrophilic "agent" or "active" or "therapeutic" or "biologic").

In an example the hydrophilic core includes nucleic acids including RNA and/or DNA and the nanoparticles are stabilized by a linear block copolymer composed of one or more polar (hydrophilic) blocks and one or more nonpolar (hydrophobic) blocks. In this example the polar blocks anchor the stabilizing polymer to the hydrophilic core of the particle, and the nonpolar blocks face outward and provide steric stability in an non-polar, or less-polar solvent phase.

In another embodiment of this invention, the hydrophilic core can include water-soluble small molecules, proteins, peptides, nucleic acids including DNA and RNA, and/or polysaccharides. The water soluble active can have a solubility in water of greater than 0.01 mg/mL and/or a logP value of less than 3. The water soluble active can have a solubility in water of greater than 0.1 mg/mL and/or a logP value of less than 2. The water soluble active can have a solubility in water of greater than 1 mg/mL and/or a logP value of less than 1. In general, the solubility of the active in water is less important to the processing than the solubility of the active in the non-process solvent. It is important that the active has low solubility in the non-polar non-process solvent. For example, the active can have a solubility in the non-process solvent of less than 1 mg/mL, less than 0.1 mg/mL, and/or less than 0.01 mg/mL. Examples of water-soluble actives includes proteins (e.g., lysozyme and ovalbumin), polypeptides, linear polypeptides (e.g., proteins), cyclic polypeptides (e.g.,

vancomycin), branched polypeptides, glycosylated peptides (e.g., vancomycin), and other biologics and nonbiologic molecules. For example, the water soluble active can have a molecular weight of from about 100 Da, 200 Da, 500 Da, 1000 Da, 2000 Da, 5000 Da, 10000 Da, 20000 Da, and 40000 Da to about 1000 Da, 2000 Da, 5000 Da, 10000 Da, 20000 Da, 40000 Da, 100 kDa, 200 kDa, 500 kDa, 1,000 kDa and 10,000 kDa. In this embodiment the stabilizing polymer is a comb polymer composed of a polar (hydrophilic) backbone with nonpolar (hydrophobic) side chains. The hydrophilic backbone anchors the stabilizing polymer to the hydrophilic core of the particle, and the nonpolar side chains face outward and provide steric stability. The hydrophilic backbone of the comb polymer may be linear or branched.

In an embodiment of this invention, the hydrophilic backbone of the comb polymer is a polysaccharide. For example, the backbone can be cellulose, dextran, maltodextrin, dextrin, dextran sulfate, dextrin sulfate, hyaluronic acid, pectins, amylopectin, amylose, pullulan, xylan, carrageenan, chitin, chitosan, starch, or combinations or modifications of these. In an embodiment of this invention the hydrophilic backbone of the comb polymer is a poly(amino acid) such as poly(glutamic acid), poly(aspartic acid), poly(lysine), poly(lysine), poly(arginine), poly(serine), poly(threonine), poly(glutamine), poly(asparagine), poly(cysteine), or combinations or modifications of these. In an embodiment of this invention the nonpolar side chains are poly(lactic acid) (PLA), poly(lactic-co-glycolic acid) (PLGA), polycaprolactone (PCL), a polyester, a poly(ortho ester), poly[(carboxyphenoxy)propane-sebacic acid], a polyphosphoester, a polyester amide, a polyurethane, a polyvinyl acrylate, a poly(amino acid), or a hydrophobically modified polysaccharide.

In this invention, we describe a method to make nanoparticles comprising of a hydrophilic core that is stabilized by comb polymers. In a process (method) according to the invention, a stabilizing polymer is dissolved in a more polar solvent to form a process solution. For example, the polar process solvent can be, but are not limited to, water, dimethyl sulfoxide (DMSO), dimethylformamide (DMF), methanol, ethanol, N-methyl-2-pyrrolidone (NMP), tetrahydrofuran (THF), glycofurol, or mixtures thereof. In an iteration of this invention the hydrophilic active as previously described is also included in the process solution with the stabilizing polymer. In an embodiment, solutes such as salts are included in the process solvent. In another iteration of this invention, the hydrophilic active is included in a separate polar process solution which may or may not be composed of the same solvent as the copolymer process solution. In an iteration of this invention the hydrophilic active is a nucleic acid. The nucleic acid can be an oligonucleotide, RNA, mRNA, siRNA, and antisense oligonucleotide (ASO), DNA, or plasmid DNA. The nucleic acid can range in molecular weight from about 500 Da, 1000 Da, 5000 Da, and 10000 Da to $10^5$ Da, $10^6$ Da, $10^7$ Da, $10^8$ Da, $10^9$ Da, $10^{10}$ Da, $10^{11}$ Da, or $10^{12}$ Da. In a method of this invention the nucleic acid is not in the acid form. For example, in a method of this invention the nucleic acid is partially or fully neutralized with a cation such as sodium, lithium, Tris, ammonium, cesium, potassium, a quaternary amine, a tertiary amine, a secondary amine, or a primary amine salt. In a method of this invention the nucleic salt form is modified to enhance the solubility of the nucleic acid in the polar process solvent.

The process solution is mixed with a more nonpolar solvent (non-process stream). For example, the non-polar non-process solvent can be, but are not limited to, chloroform, dichloromethane (DCM), tetrahydrofuran (THF), acetone, ethyl acetate, or mixtures thereof. In some embodiments of this invention there can be more than one non-process streams. Upon mixing the process and non-process stream, the hydrophilic active rapidly precipitates, becoming the core of the nanoparticle. The hydrophilic blocks of the linear block copolymer or hydrophilic backbone of the comb polymer precipitates onto the nanoparticle core and the hydrophobic blocks or branches, respectively, of the stabilizing polymer form a stabilizing shell.

In an embodiment of this invention the process and non-process solutions are rapidly mixed in a continuous process. As used here, the term "rapid mixing" means the time for process solvent/non-process solvent mixing is more rapid than the assembly time of the nanoparticles, as further described in WO 2017/112828 A1, WO 2015/200054, and US Patent 8,137,699. For example, the process solution(s) can be in a process stream(s), the non-process solvent(s) can be in a non-process solvent stream(s). The process stream can be continuously combined with the non-process stream in a confined mixing volume, and/or the formed nanoparticle can exit the confined mixing volume in an exit stream. For example, the process and non-process streams can be continuously combined in a Confined Impinging Jet Mixer or a Multi-Inlet Vortex Mixer as described and defined in Liu, Y., Cheng, C., Prud'homme, R.K. and Fox, R.O., 2008. Mixing in a multi-inlet vortex mixer (MIVM) for flash nano-precipitation. Chemical Engineering Science, 63(11), pp.2829-2842; or Johnson, B.K. and Prud'homme, R.K., 2003. Chemical processing and micromixing in confined impinging jets. AIChE Journal, 49(9), pp.2264-2282.

In a method of the invention, the nonprocess solvent can be chloroform, dichloromethane, an alkane, hexane, an ether, diethyl ether, tetrahydrofuran (THF), toluene, acetone, glycofurol or combinations. For example, the nonprocess solvent can be chloroform, dichloromethane, acetone, or combinations. For example, the polar process solvent and the nonprocess solvent can be miscible. In an embodiment, the process and nonprocess solvents are completely miscible at the volume ratios used in the nanoparticle formation process. In an embodiment, solutes may be added to the nonprocess solvent to enhance the precipitation of the active. Alternatively, in another embodiment, the process solvent is substantially soluble in the nonprocess solvent, where substantially soluble is defined as having 80% by volume of the process solvent miscible in the nonprocess solvent under volume ratios used in the nanoparticle formation process.

In a method of the invention, a time of mixing of the process solution with the nonprocess solvent is less than an assembly time of the nanoparticle. The hydrophilic active and the hydrophilic regions of the stabilizing polymer can have a supersaturation level in the solution ranging from 10 to 10,000.

A method of the invention includes stabilizing the nanoparticle core through crosslinking of the polymer. For example, the nanoparticle can be crosslinked during assembly. The crosslinking can occur after assembly.

A method of the invention includes stabilizing the nanoparticle shell through crosslinking of the polymer. For example, the nanoparticle can be crosslinked during assembly. The crosslinking can occur after assembly.

In a method of the invention, the nanoparticles may be coated by a second stabilizing polymer to create a hydrophilic shell. In an embodiment the coating polymer is a block copolymer composed of one or more hydrophilic and hydrophobic blocks. In an embodiment the hydrophilic block(s) of the coating polymer is poly(ethylene glycol) (PEG).

In a method of the invention, the nanoparticles may be processed to form microparticles. In an embodiment, the microparticles can be formed in an emulsion process. In another embodiment, the microparticles can be formed by spray drying. A polymer of the same or different type as the nanoparticle stabilizer can be included to impart desired functionality. The obtained powders may be processed or formulated according to techniques familiar to those skilled in the art.

## DESCRIPTION OF THE FIGURES

Figure 1: Process for making the active loaded inverse nanoparticle stabilized by a block copolymer. In this figure a diblock copolymer is shown, but the process is not limited to diblocks.

Figure 2: Process for making the active loaded inverse nanoparticle stabilized by a comb polymer. In this figure a comb polymer with a linear backbone is shown, but the process is not limited to this structure.

Figure 3: Example linear block copolymer constructs.

Figure 4: Example comb copolymer constructs in which the polar backbone is linear.

Figure 5: Example comb copolymer constructs in which the polar backbone is branched.

Figure 6: The nanoparticle core can be crosslinked through a variety of methods. The crosslinking can occur during or after nanoparticle assembly. The core can be crosslinked for both nanoparticles made with block copolymers and comb polymers.

Figure 7: The nanoparticle shell can be crosslinked through a variety of methods. The crosslinking can occur during or after nanoparticle assembly. The shell can be crosslinked for both nanoparticles made with block copolymers and comb polymers, but this figure is specifically showing a particle made with a comb polymer.

Figure 8: Size distributions from dynamic light scattering of PS-b-PAA stabilized nanoparticles containing RNA in the core from Example 1, Samples 1A-1D.

Figure 9: Size distributions from dynamic light scattering of PS-b-PAA stabilized nanoparticles containing RNA in the core using THF as the antisolvent, from Example 1, Samples 1E and 1F.

Figure 10: Absorbance spectra of the aqueous extraction of the solvent control, RNA control, and full nanoparticle formulation for Example 2. The absorbance peak at 260 nm is from the RNA. The nanoparticle extraction phase has a lower 260 nm absorbance compared to the RNA control because the PS-b-PAA stabilizer protects the encapsulated RNA from extraction into the aqueous phase.

Figure 11: Size distribution from Example 3 from dynamic light scattering of PS-b-PEG coated PS-b-PAA stabilized nanoparticles containing RNA in the core.

Figure 12: Size distributions of the PAsp-b-PLA-b-PEG stabilized RNA nanoparticles from Example 4 from dynamic light scattering in THF and after being processed into water.

Figure 13: Size distributions of the PAsp-b-PLA stabilized RNA nanoparticles from Example 5 from dynamic light scattering in DCM. Sample 5A had 5 v% water in the solvent stream and Sample 5B had 10 v% water in the solvent stream.

Figure 14: Size distributions of the PAsp-b-PLA-b-PAsp stabilized RNA nanoparticles from Example 6 from dynamic light scattering in DCM. Sample 6A has HRP in the core, Sample 6B has RNA, and Sample 6C has HRP and RNA.

Figure 15: Mixer inlet stream configuration and composition for RNA formulations using PAsp/PLA/PEG (Example 7).

Figure 16: Inverse NPs in chloroform produced by the formulations in Table 9 (Example 7).

Figure 17: Inverse nanoparticles loaded with 5kDa BD and stabilized by Dex-PLA. Formulations are given in Table 11 (Example 9).

Figure 18: Inverse nanoparticles loaded with 20kDa BD and stabilized by Dex-PLA. Formulations are given in Table 11 (Example 9). Samples 9E, 9F, and 9G were measured after the brine extraction.

Figure 19: Particle size distributions for sodium-RNA encapsulated into Dex-PLA inverse nanoparticles immediately after assembly, after brine extraction, after precipitation and redispersion in THF, and after coating with PLA-b-PEG into water (from Example 11).

Figure 20: Particle size distributions for sodium-RNA encapsulated into Dex-PLGA inverse nanoparticles (Example 12) immediately after assembly and after coating with PLA-b-PEG into water.

Figure 21: Particle size distributions for lithium-RNA encapsulated into Dex-PLA inverse nanoparticles (Example 13) immediately after assembly and after coating with PLA-b-PEG into water.

Figure 22: Particle size distributions for polymyxin B encapsulated into Dex-PLA inverse nanoparticles (Example 14) using DCM, ethyl acetate, THF, or acetone as the non-process solvent. All size measurements were made in the respective non-process solvent.

Figure 23: Particle size distributions for horseradish peroxidase (HRP) encapsulated into Dex-PLA inverse nanoparticles (Example 15) in DCM after the inverse Flash NanoPrecipitation process, and in water after coating with PLA-b-PEG.

Figure 24: Particle size distribution for RNA encapsulated in Dex-PLGA inverse nanoparticles (Example 21) in DCM. The RNA was in the sodium salt form in the process stream and was converted into the calcium salt during nanoparticle assembly.

Figure 25: Particle size distribution of liraglutide encapsulated in Dex-PLGA (10kDa dextran, 80wt% PLGA, 16kDa PLGA side chains) in DCM (Example 22, Sample 22F).

## DETAILED DESCRIPTION

Embodiments of the invention are discussed in detail below. In describing embodiments, specific terminology is employed for the sake of clarity. However, the invention is not intended to be limited to the specific terminology so selected.

*Flash NanoPrecipitation (FNP):* Flash NanoPrecipitation (FNP) is a process that combines rapid micromixing in a confined geometry of miscible solvent and antisolvent streams to effect high supersaturation of components. The resulting high supersaturation results in rapid precipitation and growth of the resulting nanoparticles. A stabilizing agent in the formulation accumulates on the surface of the nanoparticle and halts growth at a desired size. The process has been described in detail in Process and apparatuses for preparing nanoparticle compositions with amphiphilic copolymers and their use, BK Johnson, RK Prud'homme, US Patent 8,137,699, 2012. It has further been described in the review article by Saad and Prud'homme. (See D'addio, S. M.; Prud'homme, R. K., Controlling drug nanoparticle formation by rapid precipitation. Advanced drug delivery reviews 2011, 63 (6), 417-426; Johnson, B. K.; Prud'homme, R. K., Process and apparatuses for preparing nanoparticle compositions with amphiphilic copolymers and their use. Google Patents: 2012; Saad, W. S.; Prud'homme, R. K., Principles of nanoparticle formation by flash nanoprecipitation. Nano Today 2016, 11 (2), 212-227). In Flash NanoPrecipitation the encapsulated agents are hydrophobic and the antisolvent is more polar than the antisolvent.

The Flash NanoPrecipitation process involves a confined mixing volume having one or more solvent streams entering the mixing volume, one or more antisolvent streams entering the mixing volume, and an exit stream (leaving the mixing volume) for the process. The velocity of the inlet streams into the confined mixing volume can be between about 0.01 m/s and 100 m/s, or about 0.1 m/s and 50 m/s, or about 0.1 m/s and 10 m/s. The velocities of the streams may be equal to one another, or they may have different velocities. In the case of unequal velocities, the velocity of the highest velocity stream is the specified velocity.

*Inverse Flash NanoPrecipitation (iFNP):* Inverse Flash NanoPrecipitation (iFNP) follows the same fundamental principles as Flash NanoPrecipitation. However, in iFNP the encapsulated agents are hydrophilic, and the antisolvent is more non-polar than the solvent. The inverse Flash NanoPrecipitation process is described in: World Patents WO/2015/200054 and WO 2017/112828 A1; Pagels, R.F.; Prud'homme, R.K., Polymeric nanoparticles and microparticles for the delivery of peptides, biologics, and soluble therapeutics. J Control Release 2015, vol. 219, 519-535; Pagels, R.F.; Prud'homme, R.K., Inverse Flash NanoPrecipitation for Biologics Encapsulation: Nanoparticle Formation and Ionic Stabilization in Organic Solvents. ACS Symposium Series 2017, Vol. 1271, Chapter 11, pp 249-274; Markwalter, C.E.; Prud'homme, R.K., Inverse Flash NanoPrecipitation for Biologics Encapsulation: Understanding Process Losses via an Extraction Protocol. ACS Symposium Series 2017, Vol. 1271, Chapter 12, pp 275-296. Please note, that in some cases simply Flash Nanoprecipitation is used to refer to inverse Flash Nanoprecipitation. However, it should be clear from the encapsulated material, process solvent, and non-process solvent whether Flash NanoPrecipitation or inverse Flash NanoPrecipitation is being used.

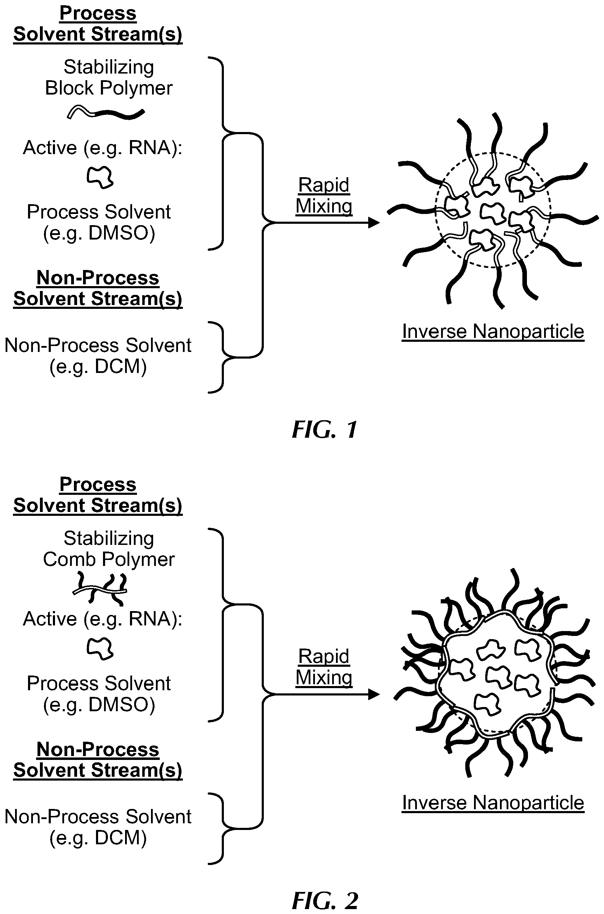

The inverse Flash NanoPrecipitation process can be used to create "inverse" particles with hydrophilic cores and/or with encapsulated water-soluble agents, such as hydrophilic peptides, proteins, and nucleic acids (Figure 1 and Figure 2). A stabilizing copolymer can be dissolved in a polar process solvent at a concentration of at least 0.1% by weight; the concentration of copolymer can be at least 0.2% by weight to form a first process solution. In an embodiment, the copolymer can be dissolved in the polar process solvent at a concentration in a range of from about 0.1 wt%, 0.2 wt%, 0.5 wt%, 1 wt%, 2 wt%, 5 wt%, 10 wt%, or 20 wt% to about 0.2 wt%, 0.5 wt%, 1 wt%, 2 wt%, 5 wt%, 10 wt%, 20 wt%, or 40 wt%. A person of skill in the art will appreciate that a factor such as the economics of a process can constrain a lower bound of concentration, and that factors such as the viscosity of the process solution or the solubility limit of the copolymer in the polar process solvent can constrain an upper bound of concentration. For example, if the viscosity of the first process solution is much greater than that of the nonprocess solvent, mixing of the first process solution with the nonprocess solvent may be inhibited. A person of skill in the art will appreciate that factors such as the molecular weight of the copolymer and the composition of the copolymer can affect the maximum concentration that can be attained in the polymer solution before the viscosity becomes too high. Examples of process solvents include, but are not limited to, water, alcohols, acetone, acetonitrile, glycol ethers, dimethyl sulfoxide (DMSO), dimethylformamide, N-methyl-2-pyrrolidone, and mixtures thereof. The process solvent can be heated or pressurized or both to facilitate dissolution of the polymer or hydrophilic active, depending on the dissolution characteristics of the copolymer in the solvent.

Upon micromixing the process solvent containing the copolymer with a less polar non-process solvent, such as chloroform, dichloromethane, or acetone, the dissimilar solubility characteristics of regions or portions of the copolymer are manifested, and the more polar portions of the copolymer can no longer exist in the soluble state, so that an "inverse" nanoparticle precipitates.

In an embodiment, additive water-soluble active agent, for example, a hydrophilic peptide, protein, or nucleic acid such as DNA or RNA, can be added to the copolymer in the process solvent. The concentration of the hydrophilic agent is typically within an order of magnitude of the concentration of the stabilizing polymer. If the concentration of the hydrophilic active is much lower than the concentration of the polymer than the final drug loading will be low. If the concentration of the hydrophilic active is much higher than the concentration of the polymer than there may not be enough stabilizing polymer to stabilize the nanoparticles. Upon creation of nanoparticles with the copolymer, the additive hydrophilic agent will be incorporated in the nanoparticle. Hydrophilic agents that are poorly soluble in the non-process solvent are coated, encapsulated, or confined as a particulate core and sterically stabilized by the protective colloid of the copolymer. The nanoparticles maintain a small and stable size in the nonprocess solvent.

In an embodiment of this invention the active agent is a nucleic acid such as and oligonucleotide, RNA, DNA, plasmid DNA, mRNA, siRNA, miRNA, or an antisense oligonucleotide. It is significant and unexpected that these highly negatively charged compounds can be encapsulated in the inverse Flash NanoPrecipitation process without the use of cationic complexation, which all other methods require (see Shin, H., et al. "Recent advances in RNA therapeutics and RNA delivery systems based on nanoparticles." Adv. Therap. 2018, 1, 180065). It was previously believed that the anionic polymer blocks (specifically poly(aspartic acid) and poly(acrylic acid)) used in the stabilizing polymers here would have an unfavorable interaction with the negatively charged nucleic acid, preventing their encapsulation. Similarly, it was thought that the lack of specific interactions between neutral polymers, such as dextran, and the nucleic acids would prevent high encapsulation efficiencies or loadings.

In another embodiment, the hydrophilic agent and copolymer are dissolved in separate process solvent streams. The process solvent used to dissolve the copolymer and the process solvent used to dissolve the hydrophilic active material may be, but are not required to be, the same. For example, the target material (water soluble agent) can be dissolved in a first polar process solvent to form a water-soluble agent solution, and the copolymer can be dissolved in a second polar process solvent to form a copolymer solution. These streams, the water-soluble agent solution and the copolymer solution, are mixed, e.g., simultaneously mixed, with the nonprocess solvent to form a mixed solution. The first polar process solvent and the second polar process solvent can be miscible, or they can be completely miscible (i.e., so that another phase is not formed) at the volumetric ratios at which they are mixed. The first polar process solvent and the nonprocess solvent can be miscible, or they can be completely miscible (i.e., so that another phase is not formed) at the volumetric ratios at which they are mixed. The second polar process solvent and the nonprocess solvent can be miscible, or they can be completely miscible (i.e., so that another phase is not formed) at the volumetric ratios at which they are mixed. In another embodiment, the hydrophilic active material and copolymer are dissolved in a single process solvent stream. This stream is then rapidly mixed with a nonprocess solvent (that is, where the time for process solvent/non-process solvent mixing is more rapid than the assembly time of the nanoparticles, as further described in WO 2017/112828 A1, WO 2015/200054, and US Patent 8,137,699).

A person skilled in the art will recognize that all solvents are miscible to some degree in each other. Miscible solvents are used in the initial nanoparticle precipitation process. "Miscible" solvents as referred to herein are those that when mixed at the ratios used in the nanoparticle formation process or the microparticle process would produce solutions that have no more than 20% of the volume of the minor phase (e.g., a polar process solvent) not dissolved in the majority phase. Completely miscible solvents as referred to herein are those that when mixed at the ratios used in the nanoparticle formation process or the microparticle process would produce solutions with no phase separation "Immiscible" solvents as referred to herein are those that when mixed at the volume ratios used in the process produce less than 20% reduction in the volume of the minor phase due to solubilization into the majority phase.

The intense micromixing of the process solution and the non-process solvent can be effected in any number of geometries. The essential idea is that high velocity inlet streams cause turbulent flow and mixing that occurs in a central cavity. The time for process solvent/non-process solvent mixing is more rapid than the assembly time of the nanoparticles. While not meant to be limiting, two such geometries have been previously described and analyzed: The Confined Impinging Jet mixer (CIJ) (Johnson, B.K., Prud'homme, R.K. Chemical processing and micromixing in confined impinging jets. AIChE Journal 2003, 49, 2264-2282; Liu, Y., Fox, R.O. CFD predictions for chemical processing in a confined impinging-jets reactor. AIChE Journal 2006, 52, 731-744) and the multi-inlet vortex mixer (MIVM) (Liu, Y., Cheng, C., Liu, Y., Prud'homme, R.K., Fox, R.O. Mixing in a multi-inlet vortex mixer (MIVM) for flash nano-precipitation. Chemical Engineering Science 2008, 63, 2829- 2842). These examples are meant to be illustrative rather than limiting or exhaustive.

The fast mixing and high energy dissipation involved in this process provide mixing timescales that are shorter than the timescale for nucleation and growth of particles, which leads to the formation of nanoparticles with active agent loading contents and size distributions not provided by other technologies. When forming the nanoparticles via Flash NanoPrecipitation, mixing occurs fast enough to allow high supersaturation levels, for example, as high as 10,000, of all components to be reached prior to the onset of aggregation. The supersaturation level is the ratio of the actual concentration of a material, for example, a copolymer, in a solvent to the saturation concentration of that material in that solvent. For example, the supersaturation levels can be at least about 1, 3, 10, 30, 100, 300, 1000, or 3000 and can be at most about 3, 10, 30, 100, 300, 1000, 3000, 10,000, 30,000, or 100,000. The timescales of aggregation of the hydrophilic active material and copolymer self-assembly are balanced. Therefore, the hydrophilic active material and polymers precipitate simultaneously, and overcome the limitations of low active agent incorporations and aggregation found with the widely used techniques based on slow solvent exchange (e.g., dialysis). The Flash NanoPrecipitation process is insensitive to the chemical specificity of the components, making it a universal nanoparticle formation technique.

The size of the resulting nanoparticles from this process can be controlled by controlling the mixing velocity used to create them, the total mass concentration of the copolymer and hydrophilic active molecules in the process solvent, the process and non-process solvents, the ratio of the copolymer and hydrophilic active molecule, and the supersaturation of the hydrophilic active molecule and non-soluble portion of the copolymer upon mixing with the non-process solvent.

Nanoparticles can be produced from copolymers that are dissolved in a process solvent with no hydrophilic active material added.

Using the methods according to the invention, particles can be made that have sizes in the range of 15 nm to 10500 nm, sizes in the range of 20 nm to 6000 nm, sizes in the range of 20 nm to 1000 nm, sizes in the range of 35 nm to 400 nm, or sizes in the range of 40 nm to 300 nm. Sizes can be determined by dynamic light scattering. For example, particles can be made that have sizes of at least about 15 nm, 20 nm, 35 nm, 40 nm, 50 nm, 100 nm, 200 nm, 300 nm, 400 nm, 600 nm, 900 nm, 1000 nm, 2000 nm, 4000 nm, or 6000 nm, and have sizes of at most about 20 nm, 35 nm, 40 nm, 50 nm, 100 nm, 200 nm, 300 nm, 400 nm, 600 nm, 900 nm, 1000 nm, 2000 nm, 4000 nm, 6000 nm, or 10500 nm. Sizes reported and cited herein are the intensity average reported values as determined by the Malvern Nanosizer deconvolution program for particles smaller than 2000 nm, and determined by scanning electron microscopy, or optical microscopy and image analysis using Image J for sizes greater than 2000 nm. Other intensity weighted deconvolution methods can be used to determine sizes of the nanoparticles.

It was previously believed that inverse Flash NanoPrecipitation required the use of stabilizing block copolymers comprising distinct blocks or domains of hydrophobic separated from blocks or domains of hydrophilic blocks or domains. These were described and presented in the patent examples in WO 2017/112828 A1 and WO 2015/200054 as di-block copolymers and tri-block copolymers. With these block copolymers, the more hydrophilic/polar block(s) precipitated onto the nanoparticle surface, and the hydrophobic blocks remained soluble in the non-process solvent and form a steric shell. In WO 2015/200054, examples employed a diblock copolymer of poly(n-butyl acrylate)-b-poly(acrylic acid). Other polymers that have been used include poly(styrene)-b-poly(acrylic acid), poly(lactic acid)-b-poly(aspartic acid), poly(lactic acid-co-glycolic acid)-b-poly(aspartic acid), and poly(ethylene glycol)-b-poly(lactic acid)-b-poly(aspartic acid).

Highly branched polymers, such as comb polymers, have a propensity to form unimolecular micelles in solvents that are antisolvents for the backbone of the comb polymer and solvents for the branches (see Fan, X., et al. "Recent Development of Unimolecular Micelles as Functional Materials and Applications." Polymer Chemistry 2016, 7, 5898-5919). Under conditions where the comb backbone is insoluble and the branches are soluble, the backbone collapses while the branches for a stabilizing corona resulting in the unimolecular micelle. The micelles do not aggregate; therefore, it was believed that they could not anchor to the surface of a growing nanoparticle during the Flash NanoPrecipitation process. Therefore, the results of the present application that comb polymers in which the backbone collapse in the non-process solvent during Flash NanoPrecipitation can be successfully used to formulate stable nanoparticles that did not aggregate was unexpected.

Previously, it was assumed that in order for the nanoparticles produced by inverse Flash NanoPrecipitation to be stable to subsequent process steps, crosslinking of the nanoparticle core was necessary (see WO 2017/112828 A1 and WO 2015/200054, Pagels, R.F.; Prud'homme, R.K., Polymeric nanoparticles and microparticles for the delivery of peptides, biologics, and soluble therapeutics. J Control Release 2015, vol. 219, 519-535; Pagels, R.F.; Prud'homme, R.K., Inverse Flash NanoPrecipitation for Biologics Encapsulation: Nanoparticle Formation and Ionic Stabilization in Organic Solvents. ACS Symposium Series 2017, Vol. 1271, Chapter 11, pp 249-274.; Markwalter, C.E.; Prud'homme, R.K., Inverse Flash NanoPrecipitation for Biologics Encapsulation: Understanding Process Losses via an Extraction Protocol. ACS Symposium Series 2017, Vol. 1271, Chapter 12, pp 275-296). Here we show the surprising result that non-crosslinked nanoparticles can be processed into coated nanoparticles or microparticles without crosslinking. This allows for the use of new stabilizing polymers in which the hydrophilic region is a non-ionic and non-crosslinkable polymer such as a polysaccharide. This is especially important for active materials that are sensitive to degradation. For example, RNA is known to degrade in the presence of multivalent cations such as $Ca^{2+}$, $Zn^{2+}$, and $Mg^{2+}$ and others (see Breslow, R., Huang, D.-L., Effects of metal ions, including Mg2+ and lanthanides, on the cleavage of ribonucleotides and RNA model compounds. Proc. Natl. Acad. Sci. v88, 4080-4083, 1991). These metal cations have been commonly used to crosslink nanoparticle cores produced by inverse Flash NanoPrecipitation, so it is important that particles without these crosslinking agents can be stably processed. Nonetheless, nanoparticles containing nucleic acids or other actives may still be crosslinked. For example, the core or the shell of the nanoparticle may be crosslinked.

### *Descriptions of Actives (Encapsulated Material)*

An active is the component or material which confers the desired performance or result. This may be a pharmaceutical active (e.g., a drug, a therapeutic, or a diagnostic (e.g., tracing) material), a fragrance, a cosmetic, a pesticide, an herbicide, an ink or a dye, a molecule or composition that enables covert security labeling, or a molecule or composition that registers a change in color when undergoing some process event. In this document we use the terms hydrophilic active, hydrophilic agent, and target interchangeably.

Encapsulated actives (target molecules) must be sufficiently polar that they rapidly precipitate in the less polar non-process solvent. Molecules that do not meet these criteria may be chemically modified to increase their water solubility and propensity to precipitate in the organic non-process solvent. Examples of biologic material that may be encapsulated include, but are not limited to, peptides, proteins, DNA, RNA, saccharides, and derivatives, conjugates, and/or analogs thereof. Small molecule water soluble therapeutics and imaging agents may also be encapsulated. Soluble stabilizing agents may be encapsulated in particles to provide stability to the particle for its use or for subsequent processing steps. Any of these materials may also be co-precipitated within a single particle. Hydrophilic material may be encapsulated for the sole purpose of adding stability to the particles during post processing. For example, material with molecular weights between 100 and 10,000,000 Daltons (Da) may be encapsulated. Material with molecular weights between 250 and 10,000,000 Da may be encapsulated. Material with molecular weights between 100 and 1,000,000 Da may be encapsulated. Material with molecular weights between 250 and 1,000,000 Da may be encapsulated. Material with molecular weights between 100 and 200,000 Da may be encapsulated.

Certain encapsulated materials may be multifunctional. For example, tobramycin is cationic and can itself be crosslinked with a copolymer. Other cationic active materials, with multiple cationic residues will similarly crosslink the anionic polymer blocks.

The encapsulated material may be incorporated into the particle at a range of loadings. For example, the mass of the encapsulated material may be greater than or equal to the mass of the copolymer. For example, the concentration of the encapsulated material in the first process solution may be from about 0.1 wt%, 0.2 wt%, 0.5 wt%, 1 wt%, 2 wt%, 5 wt%, 10 wt%, or 20 wt% to about 0.2 wt%, 0.5 wt%, 1 wt%, 2 wt%, 5 wt%, 10 wt%, 20 wt%, or 40 wt%.

Here we demonstrate for the first time that the encapsulated material can be a nucleic acid, for example RNA or DNA. The active must be soluble in the process solvent stream. Common process solvents include DMSO, DMF, NMP, methanol, ethanol, and mixture thereof. In some cases, small amounts of water can be added to the process solvent to enhance the solubility of the active agent. It was unexpected that nucleic acids require specific salt forms to be soluble in these common process solvents. It was expected that the acid (protonated) form of the nucleic acids would be the most soluble in process solvents such as DMSO because other anionic polymers including poly(acrylic acid) and poly(aspartic acid) are soluble in DMSO in the protonated acid form and not in the sodium salt from. However, partial or full neutralization of the nucleic acid with sodium hydroxide, lithium hydroxide, Tris, ammonia, cesium hydroxide, potassium hydroxide, a primary amine, a secondary amine, a quaternary amine, or a tertiary amine is required to solubilize the nucleic acid in the process solvent for inverse Flash NanoPrecipitation.

### *Description of Stabilizing Polymers*

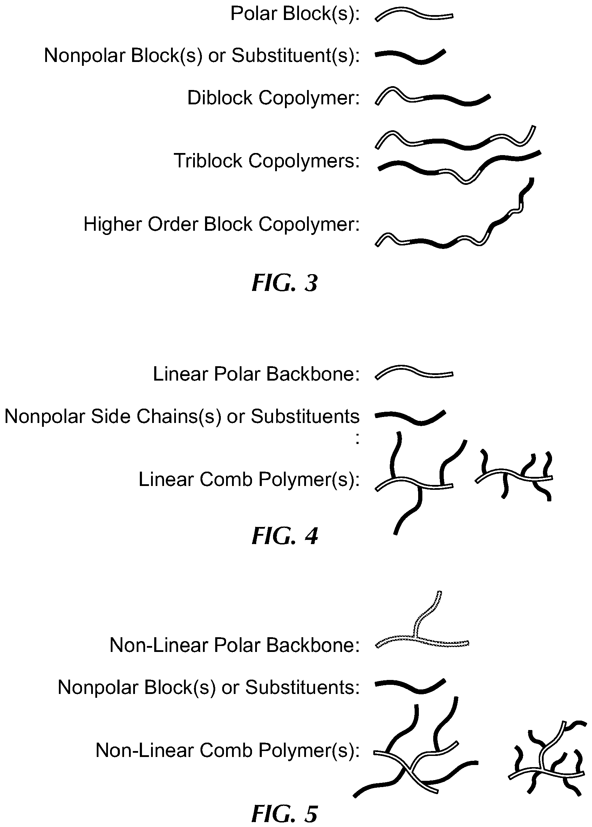

Stabilizing polymers can be linear polymers composed of one or more polar blocks and one or more nonpolar blocks (Figure 3). For example, the nonpolar block(s) can be poly(lactic acid) (PLA), poly(lactic-co-glycolic acid) (PLGA), polycaprolactone (PCL), a polyester, a poly(ortho ester), poly[(carboxyphenoxy)propane-sebacic acid], a polyphosphoester, a polyester amide, a polyurethane, a polyvinyl acrylate, a poly(amino acid), or a hydrophobically modified polysaccharide. For example, the polar block(s) can be a poly(amino acid) such as poly(glutamic acid) (PGlu), poly(aspartic acid) (PAsp), poly(lysine), poly(lysine), poly(arginine), poly(serine), poly(threonine), poly(glutamine), poly(asparagine), poly(cysteine), or combinations or modifications of these. For example, the polar block(s) can be a polysaccharide such as cellulose, dextran, maltodextrin, dextrin, dextran sulfate, dextrin sulfate, hyaluronic acid, pectins, amylopectin, amylose, pullulan, xylan, carrageenan, chitin, chitosan, starch, or combinations or modifications of these. For example, the stabilizing polymer can be PAsp-*b*-PLA, PAsp-*b*-PLGA, PAsp-*b*-PCL, PAsp-*b*-PLA-*b*-PAsp, PAsp-*b*-PLGA-*b*-PAsp, poly(ethylene glycol)-*b*-PLA-*b*-PAsp, poly(ethylene glycol)-*b*-PLGA-*b*-PAsp, poly(ethylene glycol)-*b*-PCL-*b*-PAsp, PGlu-*b*-PLA, PGlu-*b*-PLGA, PGlu-*b*-PCL, PGlu-*b*-PLA-*b*-PGlu, PGlu-*b*-PLGA-*b*-PGlu, poly(ethylene glycol)-*b*-PLA-*b*-PGlu, poly(ethylene glycol)-*b*-PLGA-*b*-PGlu, poly(ethylene glycol)-*b*-PCL-*b*-PGlu, or other combinations thereof. For example, the stabilizing polymer can be a polysaccharide-*b*-PLGA, a polysaccharide-*b*-PLA, a polysaccharide-*b*-PCL, or a higher order block copolymer composed of polysaccharide and PLA, PCL, and/or PLGA blocks.

In an embodiment of this invention the stabilizing polymer is a comb polymer consisting of a hydrophilic backbone and hydrophobic branches or grafts (Figure 4). In an embodiment of this invention the backbone of the comb polymer can be branched, like a dextran or a poly(aspartic acid) produced through condensation polymerization (Figure 5). In an embodiment of this invention the backbone of the comb polymer can be linear, like cellulose. In an embodiment of this invention, the hydrophilic backbone of the comb polymer is a polysaccharide. For example, the backbone can be cellulose, dextran, maltodextrin, dextrin, dextran sulfate, dextrin sulfate, hyaluronic acid, pectins, amylopectin, amylose, pullulan, xylan, carrageenan, chitin, chitosan, starch, or combinations or modifications of these. In an embodiment of this invention the hydrophilic backbone of the comb polymer is a poly(amino acid) such as poly(glutamic acid), poly(aspartic acid), poly(lysine), poly(lysine), poly(arginine), poly(serine), poly(threonine), poly(glutamine), poly(asparagine), poly(cysteine), or combinations or modifications of these. In an embodiment of this invention the backbone is polyvinyl alcohol. In an embodiment of this invention the nonpolar side chains are poly(lactic acid) (PLA), poly(lactic-co-glycolic acid) (PLGA), polycaprolactone (PCL), a polyester, a poly(ortho ester), poly[(carboxyphenoxy)propane-sebacic acid], a polyphosphoester, a polyester amide, a polyurethane, a polyvinyl acrylate, a poly(amino acid), or a hydrophobically modified polysaccharide. The comb polymer should consist of between 20, 30, 40, 50, 60, 70, or 80wt% and 70, 80, 90, 95wt% nonpolar side chains. The backbone of the comb polymer can be between 500 Da, 1000 Da, 2500 Da, 5000 Da, 10000 Da, or 20000 Da and 20000 Da, 50000 Da, 100000 Da, or 500000 Da. Each nonpolar side chains or grafts can be between 100, 500, 1000, 5000 Da, 10000 Da, or 20000 Da and 20000 Da, 50000 Da, 100000 Da, or 500000 Da. The comb polymer should consist of between 20, 30, 40, 50, 60, 70, or 80wt% and 70, 80, 90, 95wt% nonpolar side chains. Someone skilled in the art will recognize that the number of nonpolar side chains per polar backbone that will result in the proper wt% of nonpolar groups will depend on the molecular weights of each species. For example, the stabilizing polymer can be a dextran-graft-PLGA, dextran-graft-PLA, dextran-graft-PCL, or another polysaccharide with PLA, PCL, or PLGA grafted on. For example, the stabilizing comb polymer can be PAsp-graft-PLA, PAsp-graft-PLGA, PAsp-graft-PCL or another poly(amino acid) with PLA, PLGA, or PCL grafted on.

More generally, the stabilizing polymer can be a copolymer of one or more polar blocks coupled with one or more nonpolar blocks. The term "block" may be interpreted as either a distinct domain with a single molecular composition, or it may mean a region of the polymer chain which has regions that are predominantly more polar and other regions that are less polar. The polarity may be imparted by the monomers comprising the polymer backbone or grafted pendant groups or chains attached to the main polymer backbone. For example, the copolymer may be amphiphilic (the more nonpolar block is not water soluble), however, this is not a requirement and copolymers may be fully water soluble or fully non-water soluble, as long as solubilities of the blocks differ significantly enough in the nonprocess solvent. The copolymer should self-assemble in the nonprocess solvent, with the more polar blocks precipitating and the more nonpolar blocks remaining soluble. When used in the FNP process to make particles, the more polar blocks go to the core of the particle, and the more nonpolar blocks form a sterically protective shell. The sterically protective shell prevents particle aggregation and prevents percolation of encapsulated material during post processing steps.

Nanoparticles formed by the disclosed process can be formed with graft, block, or random copolymers. For example, these copolymers can have a molecular weight between about 1000 g/mole and about 1,000,000 g/mole, or between about 3000 g/mole and about 25,000 g/mole, or at least about 2000 g/mole.

The copolymers are comprised of repeat units or blocks that have different solubility characteristics. Typically, these repeat units are in groups of at least two comprising a block of a given character. Depending on the method of synthesis, these blocks could be of all the same repeat unit or contain different repeat units dispersed throughout the block, but still yielding blocks of the copolymer with polar and more non-polar portions. These blocks can be arranged into a series of two blocks (diblock) or three block (triblock), or more (multiblock), forming the backbone of a block copolymer. In addition, the polymer chain can have chemical moieties covalently attached or grafted to the backbone. Such polymers are graft polymers. Block units making up the copolymer can occur in regular intervals or they can occur randomly making a random copolymer. In addition, grafted side chains can occur at regular intervals along the polymer backbone or randomly making a randomly grafted copolymer. In graft polymers, polar blocks may be grafted on a non-polar polymer. More commonly, non-polar blocks or non-polar substituents are grafted on a more polar polymer chain. In graft copolymers, the length of a grafted moiety can vary. Preferably, the grafted substituents are equivalent to 2 to 22 ethylene units in length. The grafted hydrophobic substituents which create at least one less polar region of the copolymer may comprise tocopherol, tocopherol derivatives, lipids, alcohols with carbon numbers from 12 to 40, cholesterols, unsaturated and/or hydrogenated fatty acids, salts, esters or amides thereof, fatty acids mono-, di-or triglycerides, waxes, ceramides, cholesterol derivatives, or combinations. The grafted substituents may be ethyl, propyl or butyl groups commonly grafted onto saccharide monomer units to make non-polar, modified polysaccharide polymers. In addition, the grafting of the polymer backbone can be useful to enhance solvation or nanoparticle stabilization properties.

The copolymer used in the compositions and methods of the invention may be comprised of blocks of at least two repeat units or with a minimum contour length the equivalent of at least 25 ethylene units. Contour lengths are the linear sum of the polymer backbone, the molecular dimensions of which can be approximated using the Polymer Handbook, 4th Edition, eds. J. Brandrup, E.H. Immergut, and E.A. Grulke, assoc. ed. A. Abe, D.R. Bloch, 1999, New York, John Wiley & Sons.

Examples of suitable nonpolar blocks in a copolymer include but are not limited to the following: acrylates including methyl acrylate, ethyl acrylate, propyl acrylate, n-butyl acrylate (BA), isobutyl acrylate, 2-ethyl acrylate, and t-butyl acrylate; methacrylates including ethyl methacrylate, n-butyl methacrylate, and isobutyl methacrylate; acrylonitriles; methacrylonitrile; vinyls including vinyl acetate, vinylversatate, vinylpropionate, vinylformamide, vinylacetamide, vinylpyridines, vinyl phenols and vinyllimidazole; aminoalkyls including aminoalkylacrylates, aminoalkylsmethacrylates, and aminoalkyl(meth)acrylamides; styrenes; cellulose acetate phthalate, cellulose acetate succinate, hydroxypropylmethylcellulose phthalate, poly(D,L-lactide), poly (D,L-lactide-co-glycolide), poly(glycolide), poly(hydroxybutyrate), poly(alkylcarbonate) and poly(orthoesters), polyesters, poly(hydroxyvaleric acid), polydioxanone, poly(ethylene terephthalate), poly(malic acid), poly(tartronic acid), polyanhydrides, polyphosphazenes, poly(amino acids), lactic acid, caprolactone, glycolic acid, and their copolymers (see generally, Illum, L., Davids, S.S. (eds.) Polymers in Controlled Drug Delivery Wright, Bristol, 1987; Arshady, J. Controlled Release 17:1-22, 1991; Pitt, Int. J. Phar. 59:173-196, 1990; Holland et al., J. Controlled Release 4:155-0180, 1986); hydrophobic peptide-based polymers and copolymers based on poly(L-amino acids) (Lavasanifar, A., it al., Advanced Drug Delivery Reviews (2002) 54:169-190), poly(ethylene-vinyl acetate) ("EVA") copolymers, silicone rubber, polyethylene, polypropylene, polydienes (polybutadiene, polyisoprene and hydrogenated forms of these polymers), maleic anhydride copolymers of vinyl methylether and other vinyl ethers, polyamides (nylon 6,6), polyurethane, poly(ester urethanes), poly(ether urethanes), poly(esterurea). For example, polymeric blocks can include poly(ethylenevinyl acetate), poly(D,L-lactic acid) oligomers and polymers, poly(L-lactic acid) oligomers and polymers, poly(glycolic acid), copolymers of lactic acid and glycolic acid, poly(caprolactone), poly(valerolactone), polyanhydrides, copolymers of poly(caprolactone) or poly(lactic acid), or poly(propylene sulfide). For non-biologically related applications polymeric blocks can include, for example, polystyrene, polyacrylates, and butadienes.

Natural products with sufficient hydrophobicity to act as the non-polar portion of the polymer include: hydrophobic vitamins (for example vitamin E, vitamin K, and vitamin A), carotenoids, and retinols (for example, beta carotene, astaxanthin, trans and cis retinal, retinoic acid, folic acid, dihydrofolate, retinylacetate, retinyl palmintate), cholecalciferol, calcitriol, hydroxycholecalciferol, ergocalciferol, alpha-tocopherol, alpha-tocopherol acetate, alphatocopherol nicotinate, estradiol, lipids, alcohols with carbon numbers from 12 to 40, cholesterols, unsaturated and/or hydrogenated fatty acids, salts, esters or amides thereof, fatty acids mono-, di-or triglycerids, waxes, ceramides, cholesterol derivatives or mixtures thereof. For example, a natural product is vitamin E which can be readily obtained as a vitamin E succinate, which facilitates functionalization to amines and hydroxyls on the active species.

Examples of suitable polar blocks in an amphiphilic polymer that is a block copolymer include, but are not limited to the following: carboxylic acids including acrylic acid, methacrylic acid, itaconic acid, and maleic acid; polyoxyethylenes or polyethylene oxide; polyacrylamides and copolymers thereof with dimethyl-aminoethyl-methacrylate, diallyl-dimethyl-ammonium chloride, vinylbenzyl trimethylammonium chloride, acrylic acid, methacrylic acid, 2-acryamideo-2-methylpropane sulfonic acid and styrene sulfonate, polyvinyl pyrrolidone, starches and starch derivatives, dextran and dextran derivatives; polypeptides, such as polylysines, polyarginines, polyaspartic acids, polyglutamic acids; poly hyaluronic acids, alginic acids, polylactides, polyethyleneimines, polyionenes, polyacrylic acids, and polyiminocarboxylates, gelatin, and unsaturated ethylenic mono or dicarboxylic acids. To prepare anionic copolymers, acrylic acid, methacrylic acid, and/or poly aspartic acid polymers can be used. To produce cationic copolymers, DMAEMA (dimethyl aminoethyl methacrylate), polyvinyl pyridine (PVP), and/or dimethyl aminoethyl acrylamide (DMAMAM) can be used. A listing of suitable polar, water soluble, polymers can be found in Handbook of Water-Soluble Gums and Resins, R. Davidson, McGraw-Hill (1980).

The lists above of nonpolar and polar polymers should not be considered exclusive of one another. Copolymers of two polymers given in a single list may have sufficient differences in solubilities in a given nonprocess solvent to be used in this process. As an illustrative example, poly(ethylene oxide) and poly(acrylic acid) are both given in the list of polar polymers. However, poly(ethylene oxide) is soluble in chloroform and acetone, while poly(acrylic acid) is not. Therefore, copolymers of poly(ethylene oxide) and poly(acrylic acid) may be used in this process with chloroform or acetone as the nonprocess solvent.

Stabilizing random copolymers can have molecular weights ranging from about 0.1 kDa, 1 kDa, 10 kDa, 100 kDa, or 1000 kDa to about 5 kDa, 50 kDa, 100 kDa, 1000 kDa or greater.

### Description of Process and Non-Process Solvents

Formation of nanoparticles requires one or more process solvents and one or more non-process solvent streams. The process and non-process solvents may be a pure (that is, a single) liquid compound or a mixture of two or more pure liquid compounds. Other non-liquid compounds that aid in the solvent quality of the streams may be added and are also considered part of the solvent. For example, a surfactant, a salt, or a cosolvent may be added to a solvent and considered part of the solvent. These excipient compounds may or may not be in the final nanoparticle or microparticle construct, depending on the requirements of the final product.

In some cases, for example with nucleic acids, a neutralizing salt or base is required in the process solvent in order to solubilize the nucleic acid. For example, NaOH, LiOH, CsOH, KOH, Tris base, ammonium hydroxide or other basic organic or inorganic compounds can be added to help dissolve the nucleic acid. For example, a primary, secondary, quaternary, or tertiary amine can be added to solubilize the nucleic acid.

The polar process solvent containing the copolymer is chosen such that the copolymer is molecularly dissolved. This requires that the process solvent solubilize all parts of the copolymer. The process solvent containing the material to be encapsulated, if present, is also chosen such that material is molecularly dissolved. These process solvents may be, but are not required to be, the same. In some cases, both the copolymer and material to be encapsulated may be dissolved in a single solution of the process solvent. In order to dissolve the water-soluble material to be encapsulated, the process solvent is more polar than the non-process solvent. Examples of process solvents include, but are not limited to, water, alcohols, methanol, ethanol, glycol ethers, dimethyl formamide (DMF), dimethyl sulfoxide (DMSO), acetonitrile, acetone, N-methyl pyrrolidone (NMP), and mixtures thereof. Acids, bases, and salts are a few examples of additives that may be used to aid in the solubilization of the copolymer and encapsulated material in the process solvent.

The solutions of process solvent containing copolymer and material to be encapsulated are mixed with a nonprocess solvent. The non-process solvent must be capable of changing the local molecular environment of the copolymer and causing local precipitation of the hydrophilic components of the copolymer. The nonprocess solvent is chosen such that the more polar sections of the copolymer rapidly precipitate while the more non-polar components of the copolymer remain solubilized. Thus, the copolymer will self-assemble into the desired nanoparticle form in the nonprocess solvent. The nonprocess solvent is chosen such that the hydrophilic active material to be encapsulated rapidly precipitates in the final mixture. In most cases it is preferable for the process and non-process solvents to be fully miscible at the final composition. In some cases, no more than 20 volume percent of the process solvent may phase separate in the final composition. In general, this is only acceptable if the phase separated solvent goes to the core of the particles and there is no macroscopic separation. For example, some water in the process solvent may phase separate from the non-process solvent and segregate into the hydrophilic nanoparticle core. Non-process solvents include, but are not limited to, chloroform, dichloromethane, alkanes such as hexane, ethers such as diethyl ether, ethyl acetate, tetrahydrofuran (THF), toluene, acetone, and mixtures thereof. Acids, bases, and salts are a few examples of additives that may be used to aid in the precipitation of the encapsulated material and sections of the copolymer. Solvent choices are made based on the solubilities of the copolymer and encapsulated materials. It is important to note that process solvents of one system may work well as the nonprocess solvent in another system, thus the examples given above for process and nonprocess solvents should not be considered distinct.

### *Crosslinking of Nanoparticles*

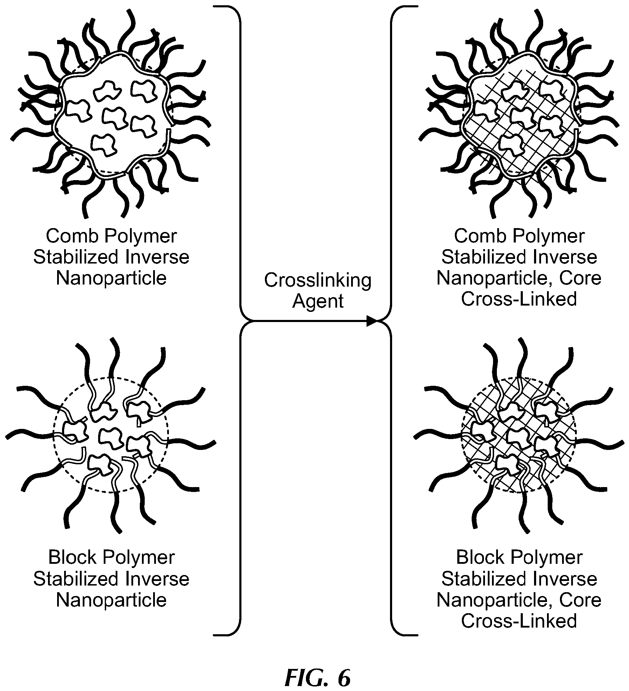

A method of the invention includes stabilizing the nanoparticle core through crosslinking of the copolymer (Figure 6). For example, the nanoparticle can be crosslinked during assembly of the nanoparticle. For example, the nanoparticle can be crosslinked after assembly of the nanoparticle. The crosslinking can be covalent crosslinking. For example, the crosslinking can be disulfide crosslinking. For example the crosslinking can occur through "Click Chemisty." The crosslinking can involve as cleavable ester linkage of the types described in USP application 13/969,449, Particulate Constructs for Release of Active Agents, Lawrence Mayer, et al. The crosslinking can be non-covalent. For example, the crosslinking can be ionic, chelation, acid-base, or hydrogen bonding crosslinking.

A crosslinking agent can be added to crosslink the copolymer. For example, the crosslinking agent can be added to crosslink groups of the copolymer having anionic functionality or character. For example, the crosslinking agent can be an alkaline earth halide, a magnesium halide, magnesium chloride, a calcium halide, calcium chloride, a transition metal halide, an iron halide, iron(III) chloride, spermine, or combinations. For example, the crosslinking agent can be a metal acetate, an alkaline earth acetate, a transition metal acetate, calcium acetate, or combinations. For example, the crosslinking agent can be chromium(III) acetate, or another chromium (III) salt. For example, the crosslinking agent can be a metal nitrate, an alkaline earth nitrate, a transition metal nitrate, calcium nitrate, zinc nitrate, iron nitrate, or combinations. Other bio-compatible multi-cationic water-soluble agents may be used as crosslinking agents, for example, to crosslink anionic sections of the copolymer. For example, the water-soluble agent can include tobramycin and the tobramycin can crosslink the copolymer. One example is tetraethylene pentamine. Ammonia or another chemical with basic character can be added to promote ionic interactions between the cationic crosslinker and the hydrophilic groups of the copolymer, if they have anionic functionality.

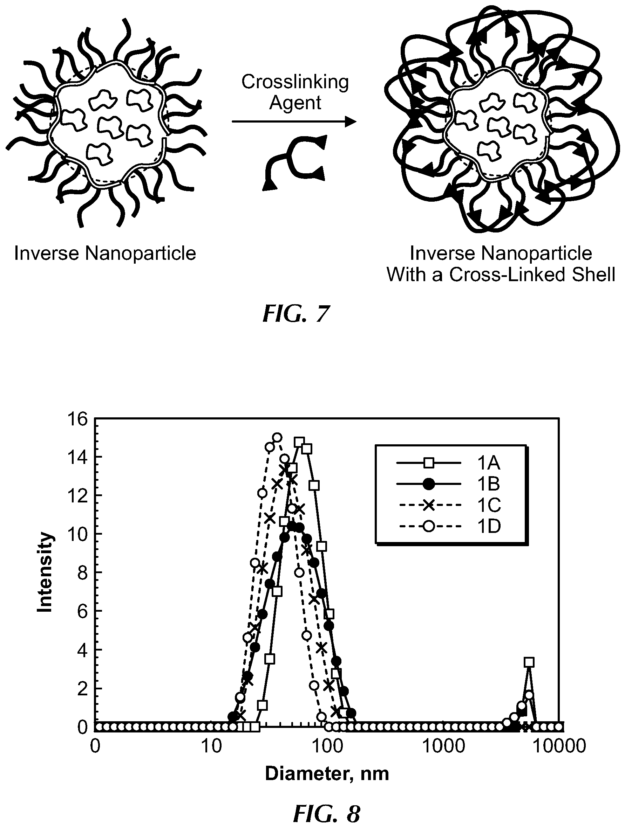

Alternatively, the shell of the nanoparticle may be crosslinked to enhance nanoparticle stability (Figure 7). For example, the end groups or other groups of the hydrophobic (non-polar) polymer chains or blocks can be crosslinked. For example, the crosslinking can be ionic or covalent. For example two or more hydrophobic polymer chains may be crosslinked directly or through the addition of a multifunctional crosslinker. For example, a hydrophobic polymer shell that contains groups with functionality "X" (on the chain end or throughout the chain) that is reactive to functionality "Y" may be crosslinked using a crosslinker that has two or more "Y" functionality groups. For example, the X and Y function groups can be thiols and acrylates, thiols and maleimides, azides and alkyenes, amines and acids, or vice versa. Functional groups X and Y can be groups that are reactive through "click chemistry." Added catalysts or other agents may be needed to induce the crosslinking of the shell.

In another method of this invention, for the first time we show that crosslinking is not required.

### *Further Processing of Inverted Nanoparticles*

After nanoparticle formation, additional processing steps can be carried out to generate a desirable formulation. Residual DMSO can be removed using an extraction process if the nanoparticles are dispersed in a water immiscible solvent. The composition of the aqueous solution can be modified to promote stability of the polymer stabilizer. For example, the aqueous stream may contain 150 mM sodium chloride to tune the osmolarity. A sugar, a PEG, or other osmolyte may be used to achieve a similar effect. The pH can be adjusted to limit stabilizer solubility. Organic acids like acetic acid or citric acid can be used, as can mineral acids such as hydrochloric acid. Similarly, bases such as ammonium hydroxide and sodium hydroxide can be added. Buffer systems such as borate or carboxylate or others may be used. The extraction can be carried out for 30 minutes or longer using standard protocols acceptable to the processing scale. The aqueous phase along with the interface is sent to waste and the organic phase is retained for further processing. This is described in Markwalter, C.E.; Prud'homme, R.K., Inverse Flash NanoPrecipitation for Biologics Encapsulation: Understanding Process Losses via an Extraction Protocol. ACS Symposium Series 2017, Vol. 1271, Chapter 12, pp 275-296.

Several different processing routes may be envisioned depending upon the formulation requirements. In Route 1, the immiscible solvent phase can be emulsified in an external aqueous phase containing a surfactant. For example, the nanoparticle phase can be emulsified in water containing poly(vinyl alcohol). Hydrophobic additives including small molecules or polymers may be added to the nanoparticle oil phase. After emulsification the solvent can be removed to form microparticles. This process is described in World Patent WO 2017/112828 A1; Pagels, R.F.; Prud'homme, R.K., Polymeric nanoparticles and microparticles for the delivery of peptides, biologics, and soluble therapeutics. J Control Release 2015, vol. 219, 519-535; Pagels, R.F.; Prud'homme, R.K., Inverse Flash NanoPrecipitation for Biologics Encapsulation: Nanoparticle Formation and Ionic Stabilization in Organic Solvents. ACS Symposium Series 2017, Vol. 1271, Chapter 11, pp 249-274.

In Route 2, the nanoparticles may be recovered directly from drying of the organic solvent to form a powder and processed by traditional routes such as tableting with additional excipients. In Route 3, the nanoparticles are spray dried (from the original organic solvent or after solvent exchange) with additional excipients to form microparticles.

Frequently, it is desirable to adjust the organic stream before proceeding through Route 1 processing. Solvent can be evaporated to generate a more concentrated nanoparticle dispersion. Additional polymer can be added as a binder, glue, or property modifier. For example, additional enteric polymers can be added before emulsification. The polymer may be the same as the nanoparticle stabilizer or it may be different. The emulsification forms a nanoparticle-in-oil-in-water system. The surfactant stabilizer can be a poly(vinyl alcohol), a pluronic, a Tween, or another stabilizer. Additives may be included in the aqueous phase such as salts, sugars, pH modifiers, osmolytes, or organic solvents to achieve specific functions. The emulsification can be carried out using techniques familiar to those skilled in the art. Removal of the residual organic phase can be achieved by evaporation under suitable conditions. The particles may be washed and lyophilized.

Route 3 is an alternative method to form microparticles that employs spray drying. Frequently, additional excipients such as additional polymer may be necessary to include. In this case, if the original organic solvent limits solubility, it may be necessary to carried out a solvent exchange to a suitable solvent such as tetrahydrofuran (THF). Crosslinking of the nanoparticle stabilizer ensures colloidal stability in this step and at the same time allows free polymer to be added prior to the spray drying step. Spray drying may be carried out using techniques that familiar to those skilled in the art.

If nanoparticles that are dispersed in water are desired, Route 4 involves coating the particles with a second stabilizing polymer such as PLA-b-poly(ethylene glycol) (PEG), PLGA-b-PEG, PCL-b-PEG, or another amphiphilic polymer. For example, the nanoparticles and stabilizer in a non-polar solvent can be mixed with a polar solvent which is a non-solvent for the nanoparticle surface and non-polar blocks of the coating polymer, causing the non-polar sections of the coating polymer to stick to the non-polar surface of the nanoparticle. In one embodiment the polar solvent is water or a mixture of water and other solvents or salts. This can involve dispersing the particles into a water-miscible solvent such as DMSO, DMF, methanol, acetone, tetrahydrofuran, ethanol, or mixtures of these. The solvent should be chosen such that it does not dissolve the nanoparticles. The nanoparticles may already be dispersed in a water-miscible solvent if water miscible solvents were used in the inverse Flash NanoPrecipitation process. Otherwise, the nanoparticles can be transferred into a water-miscible solvent through a distillation-based solvent swap, or by precipitating or drying the nanoparticles and resuspending them in a water miscible solvent. The nanoparticle dispersion and a coating polymer can then be mixed with water, a buffer, a salt solution, or a mixture of water and another water miscible solvent, in order to coat the nanoparticles. The coated nanoparticles may be freeze dried or spray dried to form a dried product.

**EXAMPLES**

*Example 1: Encapsulation of RNA with PS-b-PAA*

RNA from Torula utilis (Mr 5000-8000) was used as a model nucleic acid active and was encapsulated in polystyrene (5 kDa)-b-poly(acrylic acid) (4.8 kDa) (PS-b-PAA). RNA was dissolved at a concentration of 100 mg/mL in water with 0.5 or 1 charge equivalents of tris(hydroxymethyl)aminomethane (Tris) or ammonia with respect to the phosphate groups in the RNA. The RNA stock solution was diluted with PS-b-PAA in dimethylsulfoxide (DMSO) to produce a solution that was 5 mg/mL RNA, 5 mg/mL PS-b-PAA, and 5 v% water in DMSO. The process solution (0.5 mL) was rapidly mixed with a non-process solvent stream (0.5 mL) in a confined impinging jets (CIJ) mixer, and the effluent from the mixer was collected in a 4 mL non-process solvent bath. The non-process solvent was either chloroform (CHCl$_3$), dichloromethane (DCM), or tetrahydrofuran (THF). The specific formulations are given in Table 1. As a control, all formulations were tested without any block copolymer, which resulted in the formation of large visible RNA aggregates.

**Table 1:** The bases, equivalents of base, and non-process solvents used for the Example 1 formulations.

| Sample | Base | Base equivalents | Antisolvent |
|---|---|---|---|
| 1A | Ammonia | 1 | CHCl$_3$ |
| 1B | Tris | 1 | CHCl$_3$ |
| 1C | Tris | 1 | DCM |
| 1D | Tris | 0.5 | CHCl$_3$ |
| 1E | Tris | 1 | THF |
| 1F | Ammonia | 1 | THF |

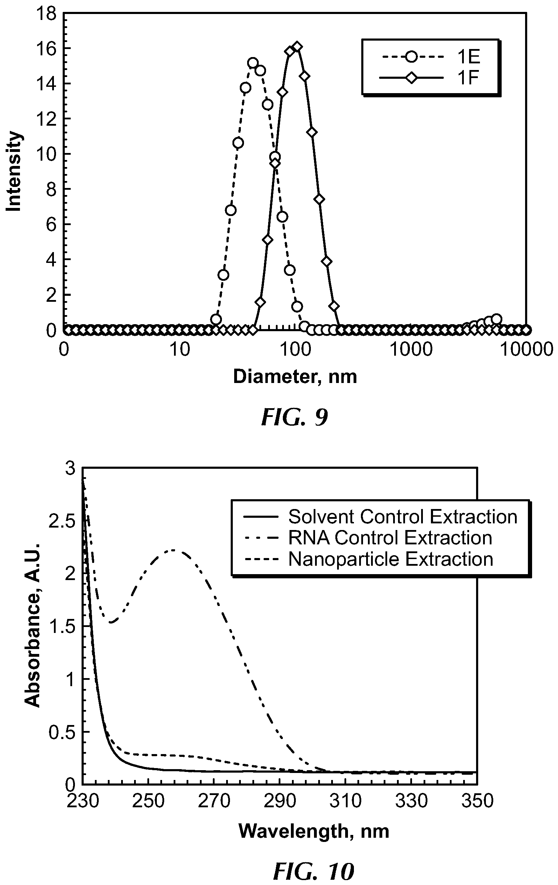

All formulations resulted in nanoparticles with no visible aggregates. Nanoparticle size distributions were measured by dynamic light scattering (DLS) in the non-process solvent used in the formulation. The PK1 diameter and the polydispersity index (PDI) from the Malvern Zetasizer DLS software are given in Table 2, and the size distributions are given in Figure 8 for Samples 1A-1D, and Figure 9 for Samples 1E and 1F.

**Table 2:** Particle diameters and PDIs for all samples in Example 1.

| Sample | PK1 Diameter, nm | PDI |
|---|---|---|
| 1A | 65 | 0.17 |
| 1B | 60 | 0.31 |
| 1C | 50 | 0.16 |
| 1D | 40 | 0.24 |
| 1E | 50 | 0.16 |
| 1F | 110 | 0.11 |

*Example 2: Metal-cation crosslinking of PS-b-PAA / RNA nanoparticles and extraction of unencapsulated RNA*

RNA from Torula utilis (Mr 5000-8000) was used as a model nucleic acid active and was encapsulated in polystyrene (5 kDa)-b-poly(acrylic acid) (4.8 kDa) (PS-b-PAA). RNA was dissolved at a concentration of 100 mg/mL in water with 0.5 charge equivalents of tris(hydroxymethyl)aminomethane (Tris) with respect to the phosphate groups in the RNA. The RNA stock solution was diluted with PS-b-PAA in dimethylsulfoxide (DMSO) to produce a solution that was 5 mg/mL RNA, 5 mg/mL PS-b-PAA, and 5 v% water in DMSO. The process solution (0.5 mL) was rapidly mixed with a CHCl$_3$ non-process solvent stream (0.5 mL) in a confined impinging jets (CIJ) mixer, and the effluent from the mixer was collected in a 4 mL CHCl$_3$ non-process solvent bath. Note that this is the same as Sample 1D in Example 1. To the final nanoparticle dispersion, 100 μL of methanol containing CaCl$_2$, ZnCl$_2$, or no salt, was added dropwise while stirring such that there was one charge equivalent of $Ca^{2+}$ or $Zn^{2+}$ to the acid groups in the PAA. The metal cations can ionically crosslink the PAA and further stabilize the nanoparticles. As controls, the above process was repeats with no block copolymer (resulting in large visible aggregates, the "RNA control") as well as with no block copolymer or RNA (just the solvents and salts, the "Solvent control").

After stirring for 30 min, 5 mL of 150 mM NaCl was added to the top of each 5 mL nanoparticle (or control) dispersion. The brine phase extracted the DMSO and any unencapsulated RNA and salts. In the case of the controls without any block copolymer, the RNA is 100% unencapsulated. The solutions were extracted for 30 mins with gentle agitation, after which the aqueous phase was removed and diluted 4-fold with water. The absorbance of the diluted aqueous phase was measured from 230 to 350 nm. The absorbance spectra for the aqueous phases of the extractions without any added metal (solvent blank without RNA or PS-b-PAA, the RNA control without the PS-b-PAA, and the RNA/PS-b-PAA nanoparticles) are given in Figure 10. It is clear from these spectra that most of the RNA is encapsulated by the PS-b-PAA block copolymer, which prevents it from being extracted into the aqueous phase. The absorbance at 260 nm (Abs) was used to calculate the encapsulation efficiency (%EE) using the following equation:

$$\%EE = 100\% - 100\% * \frac{Abs_{nanoparticle\ extraction} - Abs_{solvent\ control\ extraction}}{Abs_{RNA\ control\ extraction} - Abs_{solvent\ control\ extraction}}$$

The encapsulation efficiencies for the RNA in the inverse nanoparticles produced by inverse Flash NanoPrecipitation are given in Table 3. The addition of metal cation crosslinkers slightly increased the %EE from ~94% to ~98%, but all the measured encapsulation efficiencies are very high.

**Table 3:** RNA encapsulation efficiencies for PS-b-PAA stabilized inverse nanoparticles with no metal, or with $CaCl_2$ or $ZnCl_2$ crosslinking.

| *Metal* | *% EE* |
|---|---|
| **None** | 93.5% |
| **$CaCl_2$** | 94.4% |
| **$ZnCl_2$** | 97.5% |

### Example 3: Coating of PS-b-PAA / RNA nanoparticles with PS-b-PEG

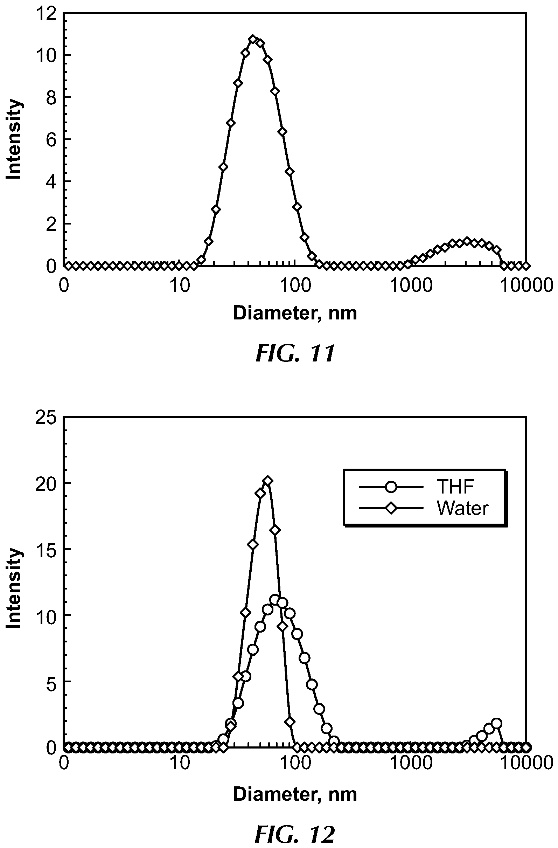

Sample 1E in Example 1, was crosslinked with $Ca^{2+}$ and coated with polystyrene(1.6 kDa)-b-poly(ethylene glycol)(5 kDa) (PS-b-PEG). Methanol (100 μL) containing $CaCl_2$ (1 charge equivalent of $Ca^{2+}$ with respect to the PAA) was added dropwise to a stirring dispersion of nanoparticles in THF. The nanoparticles were allowed to crosslink for 30 mins. After crosslinking, the nanoparticles were diluted with a solution of PS-b-PEG in THF to produce a final solution that was 1 mg/mL nanoparticles (0.5 mg/mL PS-b-PAA and 0.5 mg/mL RNA) and 1 mg/mL PS-b-PEG. This solution was rapidly mixed with an equal volume of water in a CIJ mixer and collected in a water bath such that the final solvent composition was ~25% THF and ~75% water. The size distribution of the coated nanoparticle dispersion was measured by dynamic light scattering (DLS) in water. The PK1 diameter was ~50 nm and the polydispersity index (PDI) was 0.3 from the Malvern Zetasizer DLS software. The size distribution is given in Figure 11. No visible aggregates were present, and the broad peak > 1000 nm is from air bubbles in the sample.

### Example 4: Encapsulation of RNA with PAsp-b-PLA-b-PEG

RNA from Torula utilis (Mr 5000-8000) was used as a model nucleic acid active and was encapsulated in poly(aspartic acid) (5 kDa)-b-poly(lactic acid) (10 kDa)-b-poly(ethylene glycol) (5 kDa) (PAsp-b-PLA-b-PEG). RNA was dissolved at a concentration of 100 mg/mL in water with 1 charge equivalent of tris(hydroxymethyl)aminomethane (Tris) with respect to the phosphate groups in the RNA. The RNA stock solution was diluted with PAsp-b-PLA-b-PEG in dimethylsulfoxide (DMSO) to produce a solution that was 5 mg/mL RNA, 10 mg/mL polymer and 5 v% water in DMSO. The process solution (0.5 mL) was rapidly mixed with a non-process solvent stream (THF, 0.5 mL) in a confined impinging jets (CIJ) mixer, and the effluent from the mixer was collected in a 4 mL THF non-process solvent bath. The THF precipitates the RNA and the PAsp block of the triblock copolymer, while the PLA and PEG blocks remain soluble. The nanoparticle size distribution was measured by dynamic light scattering (DLS) in THF. The PK1 diameter and the polydispersity index (PDI) from the Malvern Zetasizer DLS software are given in Table 4, and the size distributions are given in Figure 12.

**Table 4:** Particle diameters and PDIs for the PAsp-b-PLA-b-PEG stabilized RNA nanoparticles, both in THF and after crosslinking and coating in water.

| Solvent | PK1 Diameter, nm | PDI |
|---------|------------------|-----|
| THF | 80 | 0.3 |
| Water | 55 | 0.4 |

After formation, 100 $\mu$L of a 2.85 mg/mL solution of ammonium hydroxide in methanol was added dropwise to the stirring nanoparticle solution (0.75 equivalents of ammonia with respect to the acid groups of the PAsp). Subsequently, 100 $\mu$L of CaCl$_2$ in methanol was added to the stirring nanoparticles such that the final solution had 1.2 charge equivalents of Ca$^{2+}$ (0.6 molar equivalents) to the acid groups of the PAsp. The particles were crosslinked for 30 minutes. The crosslinked nanoparticles were then rapidly mixed with an equal volume of water in a CIJ mixer, and the effluent was collected in a water bath such that the final solution was ~75% water. The water collapses the inner PLA block. The nanoparticle size distribution was measured by dynamic light scattering (DLS) in water. The PK1 diameter and the polydispersity index (PDI) from the Malvern Zetasizer DLS software are given in Table 4, and the size distributions are given in Figure 12. The particles are smaller because in water than in THF because, in water, the PLA inner block collapses and forms a vitreous shell around the RNA/PAsp core. The PDI is higher mainly as an artifact of the low scattering intensity due to a small difference in refractive index between the particles and the water.

***Example 5: Encapsulation of RNA with PAsp-b-PLA***

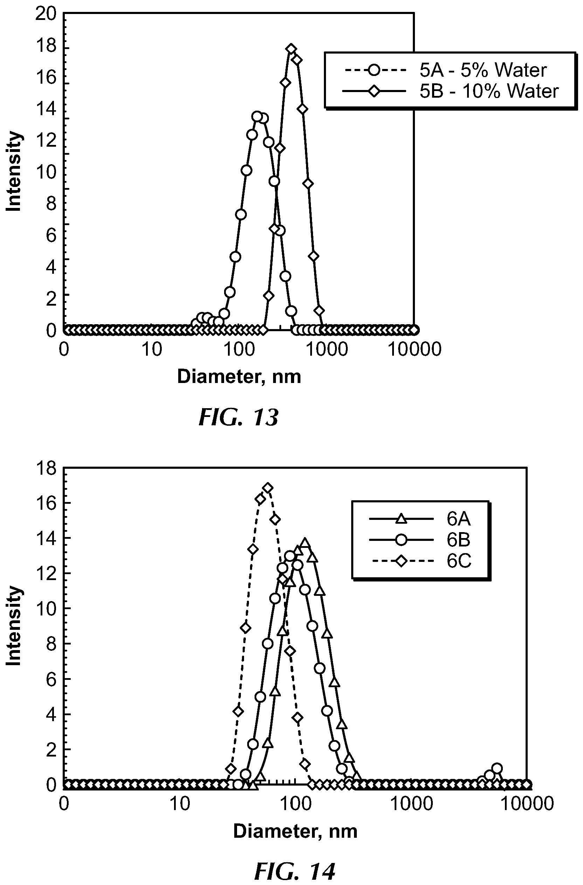

RNA from Torula utilis (Mr 5000-8000) was used as a model nucleic acid active and was encapsulated in poly(aspartic acid) (2 kDa)-b-poly(lactic acid) (12 kDa). RNA was dissolved at a concentration of 100 mg/mL in water with 1 charge equivalent of tris(hydroxymethyl)aminomethane (Tris) with respect to the phosphate groups in the RNA. The RNA stock solution was diluted with PAsp-b-PLA in dimethylsulfoxide (DMSO) to produce a solution that was 5 mg/mL RNA, 10 mg/mL polymer and 5 v% water (Sample 5A) or 10 v% water (Sample 5B) in DMSO. The process solution (0.5 mL) was rapidly mixed with a non-process solvent stream (DCM, 0.5 mL) in a confined impinging jets (CIJ) mixer, and the effluent from the mixer was collected in a 4 mL DCM non-process solvent bath. The nanoparticle size distribution was measured by dynamic light scattering (DLS) in DCM. The PK1 diameter and the polydispersity index (PDI) from the Malvern Zetasizer DLS software are given in Table 5, and the size distributions are given in Figure 13. The nanoparticle size increased going from 5 to 10 v% water in the process solvent stream because the water becomes part of the hydrophilic core.

**Table 5:** Particle diameters and PDIs for the PAsp-b-PLA-b-PEG stabilized nanoparticles in THF and after crosslinking and mixing with water.

| Sample | PK1 Diameter, nm | PDI |
|--------|------------------|-----|
| 5A - 5% water | 180 | 0.16 |
| 5B - 10% water | 430 | 0.25 |

***Example 6: Co-encapsulation of RNA and a model protein with PAsp-b-PLA-b-PAsp***

RNA from Torula utilis (Mr 5000-8000) was used as a model nucleic acid active and horse radish peroxidase (HRP) was used as a model protein. RNA and HRP were encapsulated in poly(aspartic acid) (5 kDa)-b-poly(lactic acid) (10 kDa)-poly(aspartic acid) (5 kDa) (PAsp-b-PLA-b-PAsp). The DMSO process solvent stream compositions are given in Table 6. The control (6D-6G) contained no stabilizing polymer. The RNA in all formulations was neutralized with 1 equivalent of tris(hydroxymethyl)aminomethane (Tris) with respect to the phosphate groups in the RNA. The process solution (0.5 mL) was rapidly mixed with a non-process solvent stream (DCM, 0.5 mL) in a confined impinging jets (CIJ) mixer, and the effluent from the mixer was collected in a 4 mL DCM non-process solvent bath. There were no visible aggregates in Samples 6A-6C, and the nanoparticle size distributions were measured by dynamic light scattering (DLS) in DCM. The PK1 diameter and the polydispersity index (PDI) from the Malvern Zetasizer DLS software are given in Table 7, and the size distributions are given in Figure 14. Samples 6D-6F resulted in large aggregates, as expected.

**Table 6:** Solvent stream compositions for the nanoparticle formulations produced in Example 6.

| Sample | HRP, mg/mL | RNA, mg/mL | Polymer, mg/mL | v% water in process solvent |
|--------|-----------|-----------|----------------|------------------------------|
| 6A | 5 | 0 | 5 | 5 |

(continued)

| Sample | HRP, mg/mL | RNA, mg/mL | Polymer, mg/mL | v% water in process solvent |
|---|---|---|---|---|
| 6B | 0 | 5 | 5 | 5 |
| 6C | 5 | 5 | 5 | 10 |
| 6D (control for 6A) | 5 | 0 | 0 | 5 |
| 6E (control for 6B) | 0 | 5 | 0 | 5 |
| 6F (control for 6C) | 5 | 5 | 0 | 10 |
| 6G (solvent control) | 0 | 0 | 0 | 5 |

**Table 7:** Particle diameters and PDIs for the PAsp-b-PLA-b-PAps stabilized nanoparticles in DCM with HRP, RNA, and HRP+RNA in the core.

| Sample | PK1 | PDI |
|---|---|---|
| 6A | 120 | 0.15 |
| 6B | 95 | 0.20 |
| 6C | 60 | 0.14 |

All the nanoparticles and controls were extracted with 3 mL of 150 mM NaCl in water to remove any unencapsualted HRP or RNA. The absorbance of the extracted phase was measured and the encapsulation efficiency of the RNA and HRP was calculated as given in Example 2, using the absorbance at 260 nm to measure RNA concentration and the absorbance at 400 nm to measure HRP concentration. The measured encapsulation efficiencies are given in Table 8. Note that the presence of an additional co-core material does not impact the %EE of either the RNA or the HRP.

**Table 8:** Encapsulation efficiencies of HRP and RNA in the inverse nanoparticles stabilized by PAsp-b-PLA-b-PAsp.

| Sample | HRP %EE | RNA %EE |
|---|---|---|
| 6A | 99% | n/a |
| 6B | n/a | 92% |
| 6C | 99% | 93% |

### Example 7: RNA Encapsulation into PAsp/PLA/PEG NPs

METHODS FOR EXAMPLE 7: Ribonucleic acid (RNA) from Torula utilis (Mr 5000-8000) was dissolved at 200 mg/ml in an aqueous solution of sodium hydroxide (18.7mg/ml, 0.75 eq with respect to RNA acid groups). A 25 mg/ml solution of PAsp(5 kDa)-PLA (40 kDa)-PAsp(5 kDa) in dimethyl sulfoxide (DMSO) was prepared. A similar block copolymer but with a 20 kDa PLA block was dissolved in DMSO at 12.5mg/ml. Solutions of ammonia in methanol (1.48mg/ml, NH3 basis made using a 30% ammonium hydroxide in water solution) and $CaCl_2$ dihydrate in methanol (10.67 mg/ml) were also prepared.

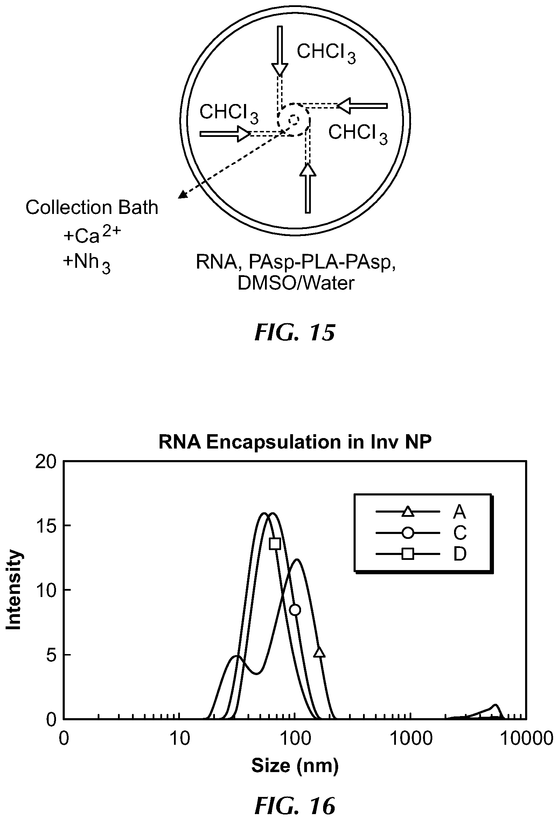

Inverse NPs containing RNA were formulated in a multi-inlet vortex mixer (Figure 15) with one solvent stream containing the formulation components and three antisolvent streams of equal flow-rate containing chloroform. The solvent stream composition were prepared from the stock solutions as noted in Table 9. The effluent was collected in a vial containing additional chloroform such that the final DMSO content was less than 10vol%. After particle assembly, 50 $\mu$L of the calcium solution was added to crosslink the PAsp residues. After 15-30 minutes of aging, 50 $\mu$L of the ammonia solution was added dropwise with rapid stirring to promote crosslinking. NPs were aged overnight at 2-8°C after dynamic light scattering (DLS) analysis.

**Table 9:** Formulations for RNA encapsulation by iFNP (DMSO process solvent stream composition)

| Sample | PLA block Mw, kDa | RNA, mg/mL | Polymer, mg/mL | v% water in process solvent | NaCl in process solvent, mM |
|---|---|---|---|---|---|
| 7A | 20 | 5 | 5 | 5 | 0 |

(continued)

| Sample | PLA block Mw, kDa | RNA, mg/mL | Polymer, mg/mL | v% water in process solvent | NaCl in process solvent, mM |
|---|---|---|---|---|---|
| 7B | 20 | 5 | 5 | 10 | 0 |
| 7C | 20 | 5 | 5 | 5 | 3.75 |
| 7D | 40 | 5 | 8.3 | 5 | 3.75 |

The residual DMSO was removed by extraction with 3 mL of 150 mM NaCl in water with mild agitation for 30 minutes. The aqueous phase was assayed for RNA losses. UV-VIS absorbance from 230 nm to 330 nm was collected for NPs batches and controls lacking either RNA or both RNA and BCP. The losses from NP formulations were reported as a fraction of the total amount extracted from the control containing only RNA, with the baseline defined by the control lacking RNA and BCP (see Example 2).

3.75mg of PLA-b-PEG were added in tetrahydrofuran (THF) to the chloroform dispersion. This organic phase containing the NP dispersion was then processed through a solvent swap using THF. The dispersion remained clear and free of aggregates. The NPs were concentrated to 5 mg/ml in THF (<5% residual $CHCl_3$) and mixed in a CIJ against either deionized water or a solution of 50 mg/ml (300 mM) PEG (MW~350g/mol) in deionized water, to act as an osmolyte balancer. The dispersion was clear and free of aggregates. It was characterized by DLS for size and zeta potential. The dispersion was filtered on a 300kDa cut-off Pall filter. EE was determined by UV-VIS analysis of either the flow-through (FT) or the NP dispersion diluted 10-fold in DMSO. The UV-VIS signal at 280nm was used to calculate the fractional content in the FT versus the total solution. Baseline was determined by diluting an aqueous solution containing the appropriate THF content.

RESULTS FOR EXAMPLE 7: Inverse NPs stabilized by $PAsp_5$-$PLA_{20}$-$PAsp_5$ were first synthesized under a range of conditions (Table 9, Figure 16). The candidate that had the narrowest size distribution and lacked aggregates (formulation "7C") contained 3.75mM NaCl in the DMSO stream (added as a higher concentration solution in water). This formulation was then prepared in triplicate using the $PAsp_5$-$PLA_{40}$-$PAsp_5$ BCP (Formulation "7D"). The BCP concentration was increased to maintain a constant mass ratio between the RNA and PAsp blocks. The triplicates of formulation D were characterized by DLS, as summarized in Table 10. While quite small, the formulation was highly stable to processing, reproducible, and was clearly differentiated from controls which contained no BCP stabilizer. In the formation of inverse NPs, these controls produced large aggregates and a cloudy solution. In the coating step, controls without the PEG BCP produced large NPs (~400 nm) with a highly negative zeta potential (-42mV).

**Table 10:** DLS analysis of RNA-encapsulated in NPs. The coated NPs are followed by the composition of the antisolvent used in their formation.

| Sample | Size (nm) | PDI | Zeta potential (mV) |
|---|---|---|---|
| 7D, inverse nanoparticle | 59 ± 3 | 0.21 ± 0.6 | n/a |
| 7D coated using deionized water | 69 ± 6 | 0.24 ± 0.03 | -12 ± 0.9 |
| 7D coated using PEG solution | 68 ± 1 | 0.25 ± 0.04 | -13 ± 1 |

The encapsulation efficiencies were measured for both the iFNP and coating steps. The inverse NPs had an EE of 95% ± 1%, calculated based on the controls without BCP. The coating step had an EE of 48% ± 7% when the antisolvent was deionized water. The use of a PEG oligomer solution (concentration 300 mM) to act as an osmotic balance to prevent NP swelling increased the EE slightly to 52% ± 3%. This concentration was likely not high enough to overcome the strong osmotic driving force generated by the high concentration of charged moieties in the NP core. This is further supported by the high agreement in DLS properties for the two antisolvent choices

### Example 8: Method for Synthesizing Dextran-branch-PLGA

Note that this is an example of how to make a comb polymer with a dextran backbone and PLA or PLGA side chains. However, the comb polymers that may be used to stabilize nanoparticles are not limited to these compositions or to being produced by this method.

Poly(lactic acid) (PLA) and poly(lactic-co-glycolic acid) acid were grafted onto a dextran backbone to produce comb polymers. In Method 1, mono-functional hydroxyl terminated PLGA is first converted to an acid terminated PLGA. 10 grams of mono-hydroxyl terminated PLGA (50:50 L:G, ~6, ~16, or ~ 24 kDa) were dried from toluene. To the PLGA 3 molar eq of 4-dimethylaminopyridine (DMAP) and 4 molar eq of succinic anhydride were added, and the mixture was dried under high vacuum. The PLGA, DMAP, and succinic anhydride were dissolved in 20mL of anhydrous DCM, and then 3 molar eq of trimethylamine (TEA) was added to the solution. The reaction proceeded for 48 hrs, after which the solution was cooled on ice and washed with cold 0.5 M HCl to remove the DMAP and TEA, followed by washes with cold water. The polymers were then precipitated three times in an excess of ice-cold isopropyl alcohol (IPA) and finally in precipitated in hexanes and dried under high vacuum. The conversion of the hydroxyl group to a carboxylic acid was verified by NMR. The polymer (1g) was dried from toluene in a tear shaped flask, after which DMAP (2 eq) and (3-Dimethylaminopropyl)-N'-ethylcarbodiimide hydrochloride (EDC, 3 eq) were added to the polymer and the mixture was dried under high vacuum. Dextran (~10 kDa) was weighed out and dried from toluene in a round bottom flask and dried under high vacuum. The mass of dextran was varied to target Dextran-branch-PLGA's with different wt% PLGA (65, 70, 75, and 80wt% PLGA for the 6kDa PLGA, 75, 80, 85, and 90wt% PLGA for the 16kDa PLGA, and 80, 85, 90, and 92.5wt% PLGA for the 24kDa PLGA). After drying, 10mL of anhydrous DMSO was added to the dextran, and 20mL of anhydrous DMSO was added to the PLGA/DMAP/EDC. Once all material were dissolved, the PLGA solution was added dropwise to the stirring dextran solution under argon. The esterification between the acid ends of the PLGA and hydroxyl groups of the dextran was allowed to proceed for 5 days. Upon completion, the reaction was diluted 4x with DCM and cooled on ice. The dextran did not crash out in the DCM, indicating that the PLGA had been conjugated. The solution was washed 3x with ice-cold 0.1 M HCl, followed by three washes with ice-cold water. The DCM phase containing the polymer was dried with magnesium sulfate and filtered through a 0.2 micron filter to remove the excess magnesium sulfate. The polymer was then concentrated, precipitated in hexanes, and dried under high vacuum.

In Method 2 the same procedure was followed, however the starting material was a symmetric, di-hydroxyl terminated HO-PLA-SS-PLA-OH. The total PLA molecular weight was 20kDa, and results in two 10kDa PLA chains when the disulfide is cleaved. The hydroxyl end groups of the PLA were converted to carboxylic acids through the ring opening of succinic anhydride as given in Method 1. The acid groups on the PLA were then conjugated to a 10kDa dextran through an EDC-mediated esterification as given in Method 1, and purified as given in Method 1. 400 mg of the final polymer was 80wt% PLA, 20wt% dextran. The polymer was dissolved in 20mL of dry $CHCl_3$ with 100μL of tributylphosphine to reduce the disulfide. After two hours, the polymer was precipitated in an excess of a dry-ice-cold mixture of hexanes and diethylether (1: 1) three times. The polymer was dried under high vacuum overnight to remove any residual solvent. In an inverse nanoparticle with a PLA corona, the thiol groups on the end of the PLA chains can be crosslinked to stabilize the nanoparticles during subsequent processing steps. For example, the thiols could be crosslinked using a polymer or small molecule with multiple acrylate groups (like a triacrylate).

### Example 9: Encapsulation and processing of Blue Dextran using Dextran-branch-PLA

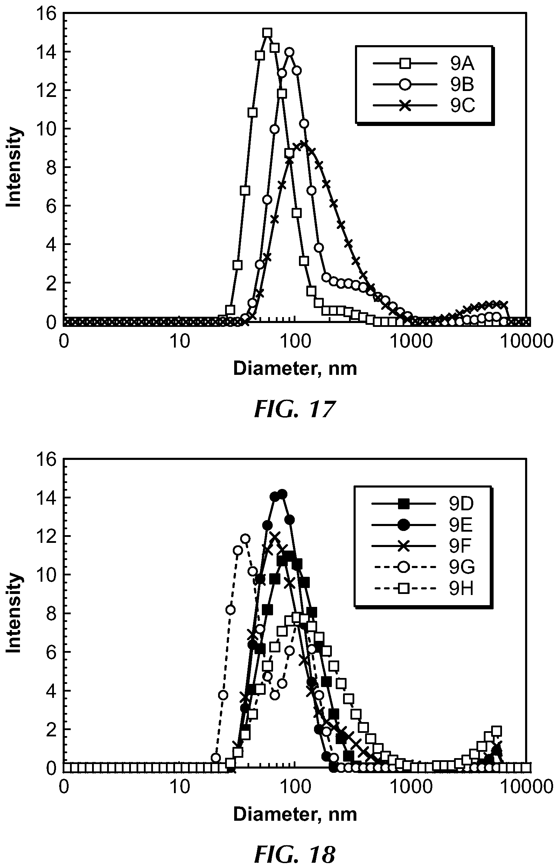

Blue dextran (BD, 5 kDa or 20 kDa) was encapsulated into inverse nanoparticles stabilized by Dextran-branch-PLA (10 kDa dextran, 10 kDa PLA side chains, 80wt% PLA) (Dex-PLA). The BD and Dex-PLA were dissolve in DMSO at the concentrations given in Table 11. In some cases small amounts of water were included in the DMSO process solvent (see Table 11). The process solution (0.5 mL) was rapidly mixed with a non-process solvent stream (DCM or $CHCl_3$ as given in Table 11, 0.5 mL) in a confined impinging jets (CIJ) mixer, and the effluent from the mixer was collected in a 4 mL non-process solvent bath (DCM or $CHCl_3$). There were no visible aggregates in Samples 9A-9H, and the nanoparticle size distributions was measured by dynamic light scattering (DLS) in DCM. The PK1 diameter and the polydispersity index (PDI) from the Malvern Zetasizer DLS software are given in Table 12, and the size distributions are given in Figure 17 for Samples 9A-9C, and Figure 18 for Samples 9D-9H.

**Table 11:** Formulation information for the BD inverse nanoparticle formulations.

| Sample | Dextran Mw, kDa | Dextran, mg/mL | Dex-PLA, mg/mL | v% water in process solvent | Non-process solvent |
|---|---|---|---|---|---|
| 9A | 5 | 5 | 5 | 10 | DCM |
| 9B | 5 | 5 | 12.5 | 0 | DCM |
| 9C | 5 | 5 | 12.5 | 0 | CHCl3 |
| 9D | 20 | 5 | 12.5 | 0 | DCM |
| 9E | 20 | 5 | 12.5 | 2.5 | DCM |
| 9F | 20 | 5 | 12.5 | 5 | DCM |
| 9G | 20 | 5 | 12.5 | 10 | DCM |
| 9H | 20 | 5 | 12.5 | 0 | CHCl3 |

**Table 12:** BD inverse nanoparticle diameters and PDIs. (*) indicated that the DLS measurement was taken after the brine extraction.

| Sample | PK1 diameter, nm | PDI |
|--------|------------------|-----|
| 9A | 70 | 0.20 |
| 9B | 130 | 0.24 |
| 9C | 120 | 0.31 |
| 9D | 95 | 0.28 |
| 9E* | 80 | 0.26 |
| 9F* | 110 | 0.23 |
| 9G* | 370 | 0.35 |
| 9H | 120 | 0.35 |

To the top of the nanoparticle organic phase, 3 mL of 150 mM NaCl was added to extract the DMSO (the so-called "brine extraction") by gently agitating for 30 min. After extraction, the organic phase containing the nanoparticles was removed. For Sample 9A, the inverse nanoparticles were put through an evaporative solvent swap by adding THF, concentrating by rotary evaporation at 40°C, and repeating 4 times to remove the water-insoluble DCM. For Sample 9B-9H, the nanoparticles were precipitated in an 8x excess of hexanes and recovered by centrifugation. The precipitated nanoparticles were washed with isopropyl alcohol and then redispersed in THF with poly(lactic acid) (4.6kDa)-b-poly(ethylene glycol) (5 kDa) (PLA-b-PEG). The final ratio of PLA-b-PEG:Dex-PLA:BD was 5:5:2, and the final total mass concentration (PLA-b-PEG + Dex-PLA + BD) was 24 mg/mL. The nanoparticle solution was then rapidly mixed with an equal volume of water in a CIJ mixer, and collected in a bath of water such that the final solvent mixture was 10v% THF. The nanoparticle size distribution was measured by DLS, and the PK1 diameter and PDI from the Malvern software is given in Table 13. The encapsulation efficiency was measured by separating the unencapsulated BD from the nanoparticle dispersion using a 300 kDa molecular weight cut-off (MWCO) Pall filter. Blue dextran concentrations were measured by UV-Vis. The resulting encapsulation efficiencies are given in Table 13. In general, the encapsulation efficiencies were high, on the order of 65-75%. In some cases, the PDI was high because excess PLA-b-PEG formed a separate micelle phase (~40 nm).

**Table 13:** Coated nanoparticle size and PDI, and BD %EE for the coated nanoparticles in water.

| Inverse Nanoparticle Sample | PK1 diameter in water, nm | PDI | %EE |
|-----------------------------|---------------------------|-----|-----|
| 9A | 50 | 0.20 | NT |
| 9B | 115 | 0.42 | 67% |
| 9C | 120 | 0.40 | 72% |
| 9D | 95 | 0.34 | 70% |
| 9E | 100 | 0.28 | 73% |
| 9F | 100 | 0.29 | 75% |
| 9G | 100 | 0.36 | 76% |
| 9H | 115 | 0.41 | 66% |

### Example 10: Encapsulation and processing of Blue Dextran with a THF antisolvent using Dextran-branch-PLA

Blue dextran (BD, 20 kDa) was encapsulated into inverse nanoparticles stabilized by Dextran-branch-PLA (10 kDa dextran, 10 kDa PLA side chains, 80wt% PLA) (Dex-PLA). The BD (5 mg/mL) and Dex-PLA (12.5 mg/mL) were dissolve in DMSO. The process solution (0.5 mL) was rapidly mixed with a THF non-process solvent stream in a confined impinging jets (CIJ) mixer, and the effluent was collected in an empty vial (no dilution). The nanoparticle size distribution was measured by dynamic light scattering (DLS) in THF. The PK1 diameter was 115 nm and the polydispersity index (PDI) was 0.07 from the Malvern Zetasizer DLS software.

PLA-b-PEG (4 mg) was added to the nanoparticles to produce a solution with 5 mg/mL PLA-b-PEG and 8.75 mg/mL nanoparticles (6.25 mg/mL Dex-PLA and 2.5 mg/mL BD). The solution was rapidly mixed in a CIJ mixer with an equal volume of water and collected in a water bath such that the final solvent composition was 1:1:18 THF:DMSO:water. The coated nanoparticle size distribution was measured by dynamic light scattering (DLS) in water. The PK1 diameter was 70 nm and the polydispersity index (PDI) was 0.15 from the Malvern Zetasizer DLS software. The encapsulation efficiency was measured by separating the unencapsulated BD from the nanoparticle dispersion using a 300 kDa molecular weight cut-off (MWCO) Pall filter. Blue dextran concentrations were measured by UV-Vis. The resulting encapsulation efficiency was 53%.

### Example 11: Encapsulation and processing of RNA in the sodium salt form using Dextran-branch-PLA

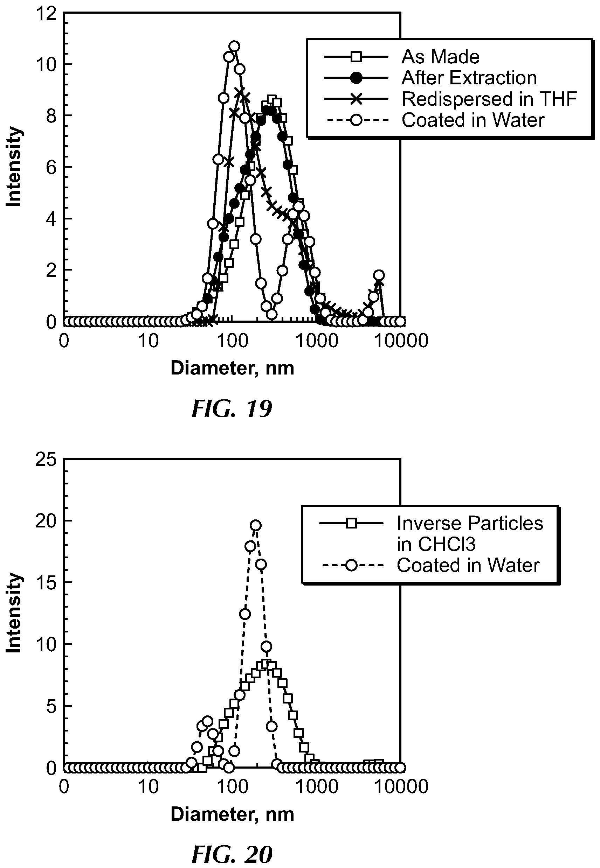

RNA from Torula utilis (Mr 5000-8000) was used as a model nucleic acid active and was encapsulated in Dex-PLA (10kDa dextran, 10kDa PLA branches, 80wt% PLA). RNA was dissolved at a concentration of 200 mg/mL in water with 0.75 or 1 charge equivalents of NaOH with respect to the phosphate groups in the RNA to form the sodium salt (see Table 14). The RNA stock solution was diluted with Dex-PLA in dimethylsulfoxide (DMSO), water, and 300 mM NaCl in water to produce a solution that was 5 mg/mL RNA, 12.5 mg/mL polymer and 5 v% water with a total NaCl concentration of 0, 3.75, or 7.5 mM (see Table 14). All formulation information is provided in Table 14. The process solution (0.5 mL) was rapidly mixed with three non-process solvent streams ($CHCl_3$, 0.5 mL each) in a multi-inlet vortex mixer (MIVM), and the effluent from the mixer was collected in a 3 mL $CHCl_3$ non-process solvent bath. The nanoparticle size distributions was measured by dynamic light scattering (DLS) in $CHCl_3$. The PK1 diameter and the polydispersity index (PDI) from the Malvern Zetasizer DLS software are given in Table 15, and the size distribution for sample 11D is given in Figure 19. Samples 11A and 11D resulted in clear solutions, while Samples 11B and 11C were hazy (Sample 11B was the haziest so a DLS measurement was not made).

**Table 14:** Formulation information for the RNA-sodium salt inverse nanoparticle formulations.

| Sample | Eq NaOH w.r.t. RNA acids | mM NaCl in DMSO with 5% MQ | Dex-PLA, mg/mL | RNA, mg/mL |
|---|---|---|---|---|
| 11A | 0.75 | 0 | 12.5 | 5 |
| 11B | 1 | 0 | 12.5 | 5 |
| 11C | 1 | 3.75 | 12.5 | 5 |
| 11D | 1 | 7.5 | 12.5 | 5 |

**Table 15:** Nanoparticle diameter (PK1) and PDI in $CHCl_3$ immediately after formation, after the brine extraction, and after being precipitated and redispersed in THF.

| | As made, in $CHCl_3$ | | After extraction, in $CHCl_3$ | | Redispersed in THF | |
|---|---|---|---|---|---|---|
| Sample | PK1, nm | PDI | PK1, nm | PDI | Pk1, nm | PDI |
| 11A | 90 | 0.24 | 90 | 0.22 | NT | NT |
| 11B | NT | NT | NT | NT | 350 | 0.38 |
| 11C | 465 | 0.47 | 320 | 0.33 | 230 | 0.24 |
| 11D | 325 | 0.29 | 280 | 0.28 | 315 | 0.40 |

The inverse nanoparticles were extracted with 3 mL of 150 mM NaCl in water to remove the DMSO and any unencapsulated RNA. The brine phase was analyzed by UV-Vis to determine the encapsulation efficiency of the initial inverse Flash NanoPrecipitation step ("iFNP % EE") which is given in Table 16. In some cases, DLS measurements were made on the nanoparticles following the extraction (Table 15). The nanoparticles were then precipitated in an 8-fold volume excess of hexanes and centrifuged from solution. Care was taken to remove as much hexanes from the pellet as possible without drying the nanoparticles. The particles were then redispersed in THF with 5 mg/mL PLA-b-PEG to produce a solution that was 5 mg/mL PLA-b-PEG and 7 mg/mL nanoparticles (2 mg/mL RNA and 5 mg/mL Dex-PLA). In some cases, DLS measurements were made on the redispersed nanoparticles in THF (Table 15). This solution was rapidly mixed with an equal volume of water in a CIJ mixer, and collected in a water bath such that the final solution was 10v% THF. Unencapsulated RNA was separated from the nanoparticles using a 300 kDa MWCO Pall filter, and the amount of encapsulated and unencapsulated RNA was assayed by UV-Vis. The encapsulation efficiency of the coating process ("Coating % EE") is given in Table 16. The nanoparticle size distribution was measured by DLS in water and the PK1 diameter and PDI are given in Table 16. Figure 19 shows Sample 10D through the entire process - as made in $CHCl_3$, post brine extraction, redispersed in THF, and coated in water.

**Table 16:** Nanoparticle diameter (PK1) and PDI after coating in water, as well as the encapsulation efficiency of the initial particle formation (iFNP %EE) from the extraction, and the encapsulation efficiency of the coating process. Overall encapsulation efficiency is multiplicative of the two processing encapsulation efficiencies.

| Sample | PK1, nm | PDI | iFNP %EE | Coating %EE |
|--------|---------|------|----------|-------------|
| 11A | 60 | 0.28 | 89% | 60% |
| 11B | 90 | 0.33 | 92% | 64% |
| 11C | 100 | 0.48 | 92% | 55% |
| 11D | 110 | 0.46 | 92% | 66% |

***Example 12: Encapsulation and processing of RNA in the sodium salt form using Dextran-branch-PLGA***

RNA from Torula utilis (Mr 5000-8000) was used as a model nucleic acid active and was encapsulated in Dex-PLGA (10kDa dextran, 16kDa PLGA branches, 80wt% PLGA). RNA was dissolved at a concentration of 200 mg/mL in water with 1 charge equivalent of NaOH with respect to the phosphate groups in the RNA to form the sodium salt. The RNA stock solution was diluted with Dex-PLA in dimethylsulfoxide (DMSO) and 300 mM NaCl in water to produce a solution that was 5 mg/mL RNA, 12.5 mg/mL polymer and 5 v% water with a total NaCl concentration of 7.5 mM. The process solution (0.5 mL) was rapidly mixed with three non-process solvent streams ($CHCl_3$, 0.5 mL each) in a multi-inlet vortex mixer (MIVM), and the effluent from the mixer was collected in a 3 mL $CHCl_3$ non-process solvent bath. The nanoparticle size distributions were measured by dynamic light scattering (DLS) in $CHCl_3$. The PK1 diameter was 260 nm and the polydispersity index (PDI) was 0.27 from the Malvern Zetasizer DLS software. The size distribution for the nanoparticles in $CHCl_3$ is given in Figure 20.

The inverse nanoparticles were extracted with 3 mL of 150 mM NaCl in water to remove the DMSO and any unencapsulated RNA. The brine phase was analyzed by UV-Vis to determine the encapsulation efficiency of the initial inverse Flash NanoPrecipitation step ("iFNP % EE"), which was found to be 88% (12% of the RNA was extracted). The nanoparticles were then precipitated in an 8-fold volume excess of hexanes and centrifuged from solution. Care was taken to remove as much hexanes from the pellet as possible without drying the nanoparticles. The particles were then redispersed in THF with 5 mg/mL PLA-b-PEG to produce a solution that was 5 mg/mL PLA-b-PEG and 7 mg/mL nanoparticles (2 mg/mL RNA and 5 mg/mL Dex-PLGA). This solution was rapidly mixed with an equal volume of water in a CIJ mixer, and collected in a water bath such that the final solution was 10v% THF. Unencapsulated RNA was separated from the nanoparticles using a 300 kDa MWCO Pall filter, and the amount of encapsulated and unencapsulated RNA was assayed by UV-Vis. The encapsulation efficiency of the coating process ("Coating % EE") was found to be 66%. The nanoparticle size distribution was measured by DLS in water and the PK1 diameter was 190 nm and PDI was 0.5. Figure 20 shows the particle size distribution after coating. The high PDI is due to the presence of a significant PLA-b-PEG micelle population.

***Example 13: Encapsulation and processing of RNA in the lithium salt form using Dextran-branch-PLA***

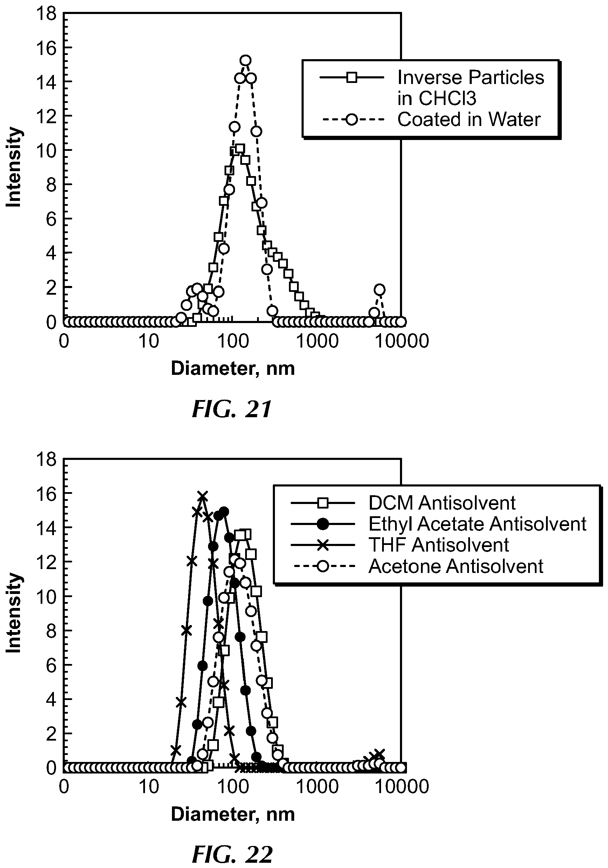

RNA from Torula utilis (Mr 5000-8000) was used as a model nucleic acid active and was encapsulated in Dex-PLA (10kDa dextran, 10kDa PLA branches, 80wt% PLA). RNA was dissolved at a concentration of 200 mg/mL in water with 1 charge equivalent of LiOH with respect to the phosphate groups in the RNA to form the lithium salt. The RNA stock solution was diluted with Dex-PLA in dimethylsulfoxide (DMSO) and 300 mM LiCl in water to produce a solution that was 5 mg/mL RNA, 12.5 mg/mL polymer and 5 v% water with a total LiCl concentration of 7.5 mM. The process solution (0.5 mL) was rapidly mixed with three non-process solvent streams ($CHCl_3$, 0.5 mL each) in a multi-inlet vortex mixer (MIVM), and the effluent from the mixer was collected in a 3 mL $CHCl_3$ non-process solvent bath. The nanoparticle size distribution was measured by dynamic light scattering (DLS) in $CHCl_3$. The PK1 diameter was 190 nm and the polydispersity index (PDI) was 0.28 from the Malvern Zetasizer DLS software. The size distribution for the nanoparticles in $CHCl_3$ is given in Figure 21.

The inverse nanoparticles were extracted with 3 mL of 150 mM NaCl in water to remove the DMSO and any unencapsulated RNA. The brine phase was analyzed by UV-Vis to determine the encapsulation efficiency of the initial inverse Flash NanoPrecipitation step ("iFNP % EE"), which was found to be 93% (7% of the RNA was extracted). The nanoparticles were then precipitated in an 8-fold volume excess of hexanes and centrifuged from solution. Care was taken to remove as much hexanes from the pellet as possible without drying the nanoparticles. The particles were then redispersed in THF with 5 mg/mL PLA-b-PEG to produce a solution that was 5 mg/mL PLA-b-PEG and 7 mg/mL nanoparticles (2 mg/mL RNA and 5 mg/mL Dex-PLA). This solution was rapidly mixed with an equal volume of water in a CIJ mixer, and collected in a water bath such that the final solution was 10v% THF. Unencapsulated RNA was separated from the nanoparticles using a 300 kDa MWCO Pall filter, and the amount of encapsulated and unencapsulated RNA was assayed by UV-Vis. The encapsulation efficiency of the coating process ("Coating % EE") was found to be 61%. The nanoparticle size distribution was measured by DLS in water and the PK1 diameter was 145 nm and PDI was 0.36. Figure 21 shows the particle size distribution after coating. The high PDI is due to the presence of a significant PLA-b-PEG micelle population.

### Example 14: Encapsulation of polymyxin B using Dextran-branch-PLA

Polymyxin B (PMB) was used as a model peptide active and was encapsulated in Dex-PLA (10kDa dextran, 10kDa PLA branches, 80wt% PLA). PMB was dissolved in DMSO at 30 mg/mL overnight. The PMB stock solution was diluted with Dex-PLA in DMSO to produce a solution that was 5 mg/mL PMB and 12.5 mg/mL polymer. No water was added to the process solvent. The process solution (0.5 mL) was rapidly mixed with a non-process solvent stream (0.5 mL) in a CIJ mixer, and the effluent from the mixer was collected in a 4 mL non-process solvent bath. The non-process solvents used were: dichloromethane (DCM), ethyl acetate, tetrahydrofuran (THF), and acetone. All non-process solvents tested resulted in a clear nanoparticle solution without the presence of visible aggregates. The nanoparticle size distributions were measured by dynamic light scattering (DLS) in the non-process solvent. The PK1 diameter and polydispersity index (PDI) from the Malvern Zetasizer DLS software are given for each formulation in Table 17. The size distributions for the nanoparticles are given in Figure 22.

Table 17: Diameter (PK1) and PDI of the PMB nanoparticles stabilized by Dex-PLA made with four different non-process solvents.

| Non-process solvent | PK1, nm | PDI |
|---|---|---|
| DCM | 150 | 0.16 |
| Ethyl acetate | 80 | 0.14 |
| THF | 50 | 0.18 |
| Acetone | 130 | 0.18 |

### Example 15: Encapsulation and processing of Horseradish Peroxidase using Dextran-branch-PLA

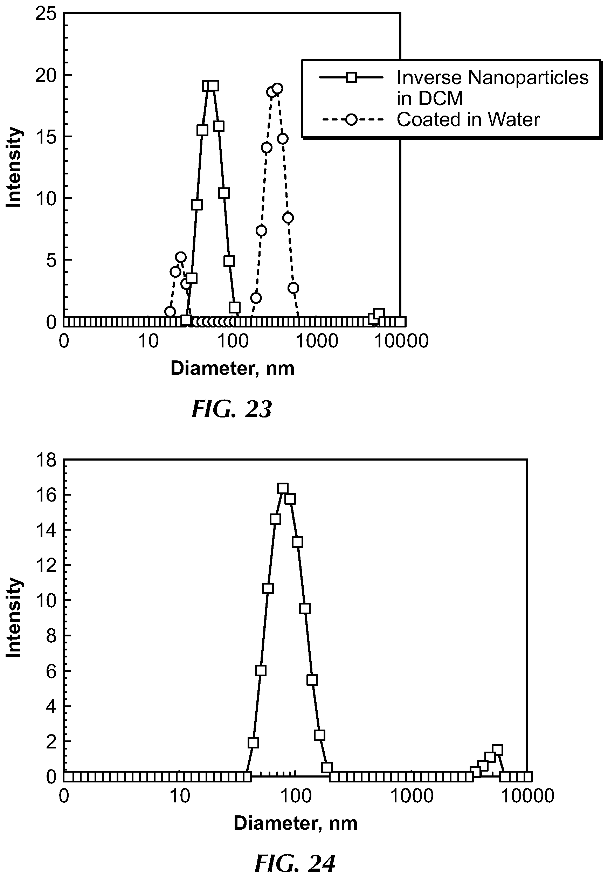

Horseradish peroxidase (HRP) was used as a model protein and was encapsulated in Dex-PLA (10kDa dextran, 10kDa PLA branches, 80wt% PLA). HRP and Dex-PLA were dissolved in dimethylsulfoxide (DMSO) with 10 v% water to produce a solution that was 5 mg/mL HRP, 12.5 mg/mL polymer and 10 v% water. The process solution (0.5 mL) was rapidly mixed with a non-process solvent stream (DCM, 0.5 mL) in a CIJ mixer, and the effluent from the mixer was collected in a 4 mL DCM non-process solvent bath. The nanoparticle size distribution was measured by dynamic light scattering (DLS) in DCM. The PK1 diameter was 60 nm and the polydispersity index (PDI) was 0.17 from the Malvern Zetasizer DLS software. The size distribution for the nanoparticles in DCM is given in Figure 23.

The inverse nanoparticles were extracted with 3 mL of 150 mM NaCl in water to remove the DMSO and any unencapsulated HRP. The organic phase retained the orange-pink color of the HRP, indicating good encapsulation. The nanoparticles were then precipitated in an 8-fold volume excess of hexanes and centrifuged from solution. The nanoparticle pellet was washed one time with isopropanol. The particles were then redispersed in acetone with 5 mg/mL PLA-b-PEG to produce a solution that was 5 mg/mL PLA-b-PEG and 7 mg/mL nanoparticles (2 mg/mL HRP and 5 mg/mL Dex-PLA). The nanoparticle size distribution was measured by DLS in water and the PK1 diameter was 330 nm and PDI was 0.5. Figure 23 shows the particle size distribution after coating. The high PDI is due to the presence of a significant PLA-b-PEG micelle population.

### Example 16: Encapsulation of 5 kDa Blue Dextran using Dextran-branch-PLA and crosslinking of shell

Blue dextran (BD, 5 kDa) was encapsulated into inverse nanoparticles stabilized by Dextran-branch-PLA (10 kDa dextran, 10 kDa PLA side chains, 80wt% PLA) (Dex-PLA) in which the PLA was terminated with thiol groups using the same formulation and method as Sample 9A in Example 9. After nanoparticle assembly, the PLA shell was crosslinked using a triacrylate. Ethoxylated (6) trimethylolpropane triacrylate (triacrylate-6, molecular weight 560 Da, containing three acrylate groups, each linked to trimethylolpropane by two ethylene glycol repeat units) was dissolved in DMSO at a concentration of 1mg/mL. 15 μL of the triacylate-6 solution was added to 2 mL of the nanoparticle solution, resulting in a 1:1 molar ratio of acrylate groups to thiol groups. To catalyze the reaction, either 1 eq of triethylamine (TEA), 1 eq of DMAP, 0.2 eq of tributylphosphine, or 0.2 eq of hexyl-amine was added to the nanoparticles (note: all equivalents are with respect to the thiol groups). A control nanoparticle solution contained no added catalyst. The nanoparticle dispersions were mixed for 48 hrs to allow the crosslinking to occur. After 48 hrs, 1 mL of 150 mM NaCl in water was added to the top of each nanoparticle dispersion. The nanoparticle dispersions with added catalysts remained clear, while the dispersion without added catalyst became more opalescent/hazy. Without catalyst the nanoparticles can swell in the presence of water (hence the more opaque solution), while crosslinking prevents the particles from swelling.

### Example 17: Encapsulation of 20 kDa Blue Dextran using Dextran-branch-PLA and crosslinking of shell

Blue dextran (BD, 20 kDa) was encapsulated into inverse nanoparticles stabilized by Dextran-branch-PLA (10 kDa dextran, 10 kDa PLA side chains, 80wt% PLA) (Dex-PLA) in which the PLA was terminated with thiol groups using the same formulation and method as Sample 9D in Example 9. After nanoparticle assembly, the PLA shell was crosslinked using a triacrylate. Ethoxylated (6) trimethylolpropane triacrylate (triacrylate-6, molecular weight 560 Da, containing three acrylate groups, each linked to trimethylolpropane by two ethylene glycol repeat units) was dissolved in DMSO at a concentration of 1mg/mL. Ethoxylated (15) trimethylolpropane triacrylate (triacrylate-15, molecular weight 956 Da, containing three acrylate groups, each linked to trimethylolpropane by five ethylene glycol repeat units) was dissolved in DMSO at a concentration of 1mg/mL. 75 $\mu$L of the triacylate-6 solution or 127.5 $\mu$L of the tracrylate-15 solution was added to 5 mL of the nanoparticle solution, resulting in a 1:1 molar ratio of acrylate groups to thiol groups. To catalyze 0.2 eq of tributylphosphine was added to the nanoparticles (note: all equivalents are with respect to the thiol groups). A control nanoparticle solution contained no added catalyst. The nanoparticle dispersions were mixed for 48 hrs to all the crosslinking to occur. After 48 hrs, 3 mL of 150 mM NaCl in water was added to the top of each nanoparticle dispersion. The nanoparticle dispersions with added catalysts remained clear, while the dispersion without added catalyst became more opalescent/hazy. Without catalyst the nanoparticles can swell in the presence of water (hence the more opaque solution), while crosslinking prevents the particles from swelling.

### Example 18: Encapsulation of RNA in different salt forms using Dextran-branch-PLGA

RNA from Torula utilis (Mr 5000-8000) was used as a model nucleic acid active and was encapsulated in Dex-PLGA (10kDa dextran, 16kDa PLGA branches, 80wt% PLGA). RNA was dissolved at a concentration of 200 mg/mL in water with 0.9 charge equivalents of either (1) NaOH, (2) N-acetyl histamine (NAH), (3) triethylamine (TEA), or (4) triethanolamine (TEtOHA) with respect to the phosphate groups in the RNA to form different salt forms. The RNA stock solution was diluted with Dex-PLA in dimethylsulfoxide (DMSO) and 300 mM NaCl in water to produce a solution that was 5 mg/mL RNA, 12.5 mg/mL polymer and 5 v% water with a total NaCl concentration of 7.5 mM. The process solution (0.5 mL) was rapidly mixed with three non-process solvent streams ($CHCl_3$, totaling 4.5 mL) in a multi-inlet vortex mixer (MIVM). The nanoparticle size distributions were measured by dynamic light scattering (DLS) in $CHCl_3$ (Table 18).

**Table 18:** Diameter (PK1) and PDI of the RNA-loaded nanoparticles stabilized by Dex-PLGA made with four different RNA salt forms.

| Salt Form | PK1, nm | PDI |
|---|---|---|
| NaOH | 60 | 0.27 |
| NAH | 58 | 0.18 |
| TEA | 60 | 0.29 |
| TEtOH | 60 | 0.25 |

### Example 19: Formation of RNA-calcium salt in nanoparticles stabilized with Dextran-branch-PLGA

The salt forms of the RNA-loaded nanoparticles made in Example 18 were changed by adding $Ca^{2+}$ after the nanoparticle assembly. Calcium chloride dihydrate was dissolved in methanol at a concentration of 19.4 mg/mL, and 29.4 uL of this solution was added to the stirring nanoparticle dispersion (1 charge equivalent of $Ca^{2+}$ per phosphate group in the RNA). The particles were stirred overnight to allow the calcium to interact with the RNA. The nanoparticle size distributions were measured by dynamic light scattering (DLS) in $CHCl_3$ (see Table 19, below). After adding calcium, the count rate increased in the DLS measurement, indicating greater scattering by the newly formed calcium-RNA salt (Table 19).

**Table 19:** Diameter (PK1), PDI, and DLS count rates of the RNA-loaded nanoparticles stabilized by Dex-PLGA made with four different RNA salt forms after adding $Ca^{2+}$.

| Original Salt Form | PK1, nm | PDI | Count Rate without $Ca^{2+}$ | Count Rate with $Ca^{2+}$ |
|---|---|---|---|---|
| NaOH | 105 | 0.22 | 125 | 759 |
| NAH | 110 | 0.11 | 148 | 1079 |
| TEA | 75 | 0.07 | 104 | 365 |
| TEtOH | 76 | 0.08 | 170 | 421 |

### Example 20: Encapsulation of RNA in different salt forms using Poly(aspartic acid)-b-PLGA

RNA from Torula utilis (Mr 5000-8000) was used as a model nucleic acid active and was encapsulated in PAsp-b-PLGA (2kDa poly(aspartic acid), 16kDa PLGA). RNA was dissolved at a concentration of 200 mg/mL in water with 0.9 charge equivalents of either (1) NaOH, (2) N-acetyl histamine (NAH), (3) triethylamine (TEA), or (4) triethanolamine (TEtOHA) with respect to the phosphate groups in the RNA to form different salt forms. The RNA stock solution was diluted with PAsp-b-PLGA in dimethylsulfoxide (DMSO) and 300 mM NaCl in water to produce a solution that was 5 mg/mL RNA, 12.5 mg/mL polymer and 5 v% water with a total NaCl concentration of 7.5 mM. The process solution (0.5 mL) was rapidly mixed with three non-process solvent streams ($CHCl_3$, totaling 4.5 mL) in a multi-inlet vortex mixer (MIVM). The nanoparticle size distributions were measured by dynamic light scattering (DLS) in $CHCl_3$ (Table 20).

**Table 20:** Diameter (PK1) and PDI of the RNA-loaded nanoparticles stabilized by PAsp-b-PLGA made with four different RNA salt forms.

| Salt Form | PK1, nm | PDI |
|---|---|---|
| NaOH | 110 | 0.30 |
| NAH | 175 | 0.27 |
| TEA | 75 | 0.21 |
| TEtOH | 75 | 0.21 |

After nanoparticle assembly, calcium was added to the nanoparticle dispersions to crosslink the acid groups of the poly(aspartic acid) block of the stabilizing polymer as well as the phosphate backbone of the RNA. Calcium chloride dihydrate was dissolved in methanol at a concentration of 19.4 mg/mL. This solution was then added to the stirring nanoparticle dispersions such that there was 1 charge equivalent of $Ca^{2+}$ for every acid group of the PAsp and phosphate group of the RNA. The nanoparticle dispersion was mixed for at least 30 min to allow the calcium to interact with the PAsp and the RNA.

### Example 21: In situ formation of the calcium-salt of RNA and encapsulation by Dextran-branch-PLGA

RNA from Torula utilis (Mr 5000-8000) was used as a model nucleic acid active and was encapsulated in Dex-PLGA (10kDa dextran, 16kDa PLGA branches, 80wt% PLGA). RNA was dissolved at a concentration of 200 mg/mL in water with 0.9 charge equivalents of NaOH with respect to the phosphate groups in the RNA to form the sodium salt. The RNA stock solution was diluted with Dex-PLA in dimethylsulfoxide (DMSO) and 300 mM NaCl in water to produce a 500 uL process solution that was 5 mg/mL RNA, 12.5 mg/mL polymer and 5 v% water with a total NaCl concentration of 7.5 mM. Calcium chloride dihydrate was dissolved in methanol at a concentration of 19.4 mg/mL, and 29.4 uL of this solution was added to 500 uL of chloroform to produce a non-process solvent. The process solution (500 uL), calcium-containing non-process solvent (500 uL), and excess non-process solvent (chloroform, 2 mL) were rapidly mixed in a MIVM. The final mixture had 1 charge equivalent of $Ca^{2+}$ per phosphate group in the RNA in order to form the calcium salt of the RNA during nanoparticle assembly. The nanoparticle size distribution was measured by dynamic light scattering (DLS) in $CHCl_3$. The PK1 diameter was 90 nm, and the PDI was 0.22, and the particle size distribution is given in Figure 24.

### Example 22: Encapsulation of liraglutide by Dextran-branch-PLGA with different polymer stabilizer compositions

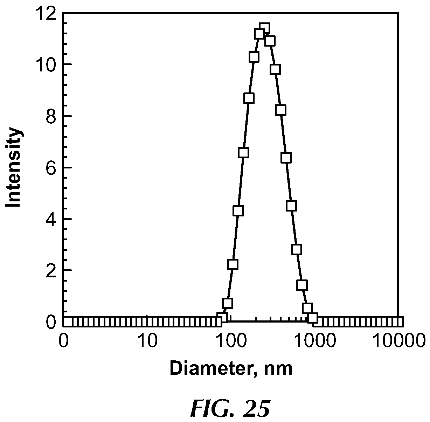

Liraglutide, a peptide therapeutic, was encapsulated into nanoparticles stabilized by Dex-PLGA with different polymer compositions. All comb polymers were composed of a 10kDa dextran backbone, with PLGA (1:1 L:G ratio) side chains. The PLGA side chains were either 6.3 kDa, 16 kDa, or 23 kDa in molecular weight. The comb polymers ranged in composition from 65 to 92.5wt% PLGA (see Table 21 below). The process solvent stream was DMSO with 10v% water with 5 mg/mL liraglutide (HCl salt), 5 mg/mL Dex-PLGA, and 15 mM NaCl. The process solution (0.5 mL) was rapidly mixed with three non-process solvent streams (dichloromethane, DCM, totaling 4.5 mL) in a multi-inlet vortex mixer (MIVM). The nanoparticle size distributions were measured by dynamic light scattering (DLS) in $CHCl_3$ (Table 20). The particle size distribution for Sample 22F is given in Figure 25.

**Table 21:** Diameter (PK1) and PDI of liraglutide-loaded nanoparticles stabilized by different Dex-PLGA comb polymers in DCM.

| Sample | PLGA Branch Mw | Wt% PLGA in the comb polymer | PK1, nm | PDI |
|--------|----------------|------------------------------|---------|-----|
| 22A | 6.3 kDa | 65 | 180 | 0.07 |
| 22B | 6.3 kDa | 70 | 250 | 0.15 |
| 22C | 6.3 kDa | 75 | 285 | 0.17 |
| 22D | 6.3 kDa | 80 | 270 | 0.18 |
| 22E | 16 kDa | 75 | 270 | 0.21 |
| 22F | 16 kDa | 80 | 290 | 0.17 |
| 22G | 16 kDa | 85 | 310 | 0.18 |
| 22H | 16 kDa | 90 | 325 | 0.21 |
| 22I | 23 kDa | 80 | 355 | 0.24 |
| 22J | 23 kDa | 85 | 300 | 0.21 |
| 22K | 23 kDa | 90 | 290 | 0.17 |
| 22L | 23 kDa | 92.5 | 315 | 0.18 |

### Example 23: Assembly of liraglutide-loaded Dex-PLGA stabilized inverse nanoparticles into microparticles.

The liraglutide-loaded nanoparticles of Sample 22F (from Example 22, above), were assembled into microparticles for depot delivery applications. First, 3 mL of 150 mM NaCl was added to the top of the nanoparticle dispersion (5 mL) and mixed gently to extract DMSO. The aqueous and organic phases were separated by centrifugation at 1000 rcf for 5 min, and the organic phase containing the nanoparticles was removed. Dextran powder was added to the nanoparticle dispersion to absorb any water, and the dextran powder was removed by filtering through a 1-micron filter. The recovery of liraglutide through this processing was measured to be about 70%. PLGA homopolymer was added to the nanoparticle dispersion at a 2:1 mass ratio to the liraglutide (2 mg PLGA for every mg of liraglutide). The nanoparticles and PLGA were concentrated to a total mass concentration of ~10 mg/mL by rotary evaporation at 35°C and 400 torr in a glass vial that had been silanized using trichloro(octadecyl) silane. This organic phase (500 uL) was added to 5 mL of an aqueous phase composed of phosphate buffered saline and 1 wt% polyvinyl alcohol. An oil-in-water emulsion was produced by vortexing the mixture at 750 rpm for 1 min. The emulsion was placed on a rocker with a stir bar, and the organic solvent was stripped at room temperature by evaporation under vacuum at 300 torr for 15 min, 250 torr for 15 min, 200 torr for 15 min, 150 torr for 15 min, 100 torr for 30 min, and 50 torr for 1 hr (total time: 2.5 hrs) to produce hardened microparticles. The microparticles were passed through a 70-micron strainer to remove any large particles, and washed with water on a 10-micron sieve to remove any small particles, fragments, or unencapsulated liraglutide. The washed particles were freeze dried. The final particles were 24.3wt% liraglutide. With a target loading of 25wt% (25 wt% liraglutide, 25wt% Dex-PLGA, and 50wt% PLGA homopolymer), this is an encapsulation efficiency of 97%.

### Claims

1. A nanoparticle comprising:
   a hydrophilic core stabilized by a comb polymer with a hydrophilic portion of the comb polymer oriented towards the center of the nanoparticle.

2. The nanoparticle according to claim 1, wherein the nanoparticle is not crosslinked, at least a portion of the hydrophilic core is crosslinked, or a hydrophobic portion of the comb polymer is crosslinked.

3. The nanoparticle according to claim 1, wherein the hydrophilic portion of the copolymer comprises a non-ionic polymer.

4. The nanoparticle according to claim 3, wherein the hydrophilic portion non-ionic copolymer is a polysaccharide.

5. The nanoparticle according to claim 1, wherein the hydrophilic core comprises a water-soluble small molecule, a protein, a peptide, a nucleic acid, a polysaccharide, or a combination thereof.

6. The nanoparticle according to claim 5, wherein the nucleic acid is RNA or DNA.

7. A method for manufacturing a nanoparticle, comprising the steps of:

forming a first process solution by dissolving a stabilizing polymer having a hydrophilic portion and a hydrophobic portion in a polar solvent stream having a first process solvent;
forming a second process solution by dissolving hydrophilic active in a polar solvent, which may be the first process solvent or a different polar solvent;
combining the hydrophilic active, stabilizing polymer, and solvent with a non-process stream or streams, where the non-process stream or streams contains an additional solvent that is more nonpolar than the solvents used in the first or second process solvent streams;
allowing the hydrophilic active to precipitate and form a hydrophilic core; and
allowing the hydrophilic portion of the stabilizing polymer to precipitate onto the hydrophilic core and the hydrophobic portion to stabilize the core.
wherein the stabilizing polymer is a comb polymer.

8. The method according to claim 7, wherein the hydrophilic active is added to a separate polar process solvent and the two process solvent streams and the non-process solvent stream are rapidly micromixed in a confined mixing volume under continuous flow conditions.

9. The method according to claim 8, wherein the separate polar process solvent is the same as the solvent.

10. The method according to claim 9, wherein the hydrophilic active is added to the solvent.

11. The method according to claim 10, wherein the hydrophilic active is a nucleic acid.

12. The method according to claim 11, wherein the nucleic acid is a nucleic acid salt.

13. The method according to claim 11, wherein the nucleic acid is combined with a neutralizing base prior to adding to the polar process solvent.


**Patentansprüche**

1. Nanoteilchen, umfassend:
einen hydrophilen Kern, der durch ein Kammpolymer mit einem hydrophilen Abschnitt des Kammpolymers, der in Richtung des Mittelpunkts des Nanoteilchens ausgerichtet ist, stabilisiert ist.

2. Nanoteilchen nach Anspruch 1, wobei das Nanoteilchen nicht vernetzt ist, mindestens ein Abschnitt des hydrophilen Kerns vernetzt ist oder ein hydrophober Abschnitt des Kammpolymers vernetzt ist.

3. Nanoteilchen nach Anspruch 1, wobei der hydrophile Abschnitt des Copolymers ein nichtionisches Polymer umfasst.

4. Nanoteilchen nach Anspruch 3, wobei der nichtionische hydrophile Abschnitt des Copolymers ein Polysaccharid ist.

5. Nanoteilchen nach Anspruch 1, wobei der hydrophile Kern ein wasserlösliches kleines Molekül, ein Protein, ein Peptid, eine Nukleinsäure, ein Polysaccharid oder eine Kombination davon umfasst.

6. Nanoteilchen nach Anspruch 5, wobei die Nukleinsäure RNA oder DNA ist.

7. Verfahren zum Herstellen eines Nanoteilchens, umfassend die folgenden Schritte:

Bilden einer ersten Prozesslösung durch Auflösen eines stabilisierenden Polymers, das einen hydrophilen Abschnitt und einen hydrophoben Abschnitt aufweist, in einem polaren Lösungsmittelstrom, der ein erstes Prozesslösungsmittel aufweist;
Bilden einer zweiten Prozesslösung durch das Auflösen eines hydrophilen Wirkstoffs in einem polaren Lösungsmittel, das das Lösungsmittel des ersten Prozesses oder ein unterschiedliches polares Lösungsmittel sein kann;
Verbinden des hydrophilen Wirkstoffs, stabilisierenden Polymers und des Lösungsmittels mit einem Nichtprozessstrom oder Nichtprozessströmen, wobei der Nichtprozessstrom oder die Nichtprozessströme ein zusätzliches Lösungsmittel enthalten, das unpolarer ist als die Lösungsmittel, die in den ersten oder zweiten Prozesslösungsmittelströmen verwendet werden;
Ermöglichen, dass der hydrophile Wirkstoff ausfällt und einen hydrophilen Kern bildet; und
Ermöglichen, dass der hydrophile Abschnitt des stabilisierenden Polymers auf den hydrophilen Kern ausfällt und der hydrophobe Abschnitt den Kern stabilisiert,
wobei das stabilisierende Polymer ein Kammpolymer ist.

8. Verfahren nach Anspruch 7, wobei der hydrophile Wirkstoff zu einem separaten polaren Prozesslösungsmittel hinzugefügt wird und die zwei Prozesslösungsmittelströme und der Nichtprozesslösungsmittelstrom in einem begrenzten Mischvolumen unter kontinuierlichen Flussbedingungen schnell mikrovermischt werden.

9. Verfahren nach Anspruch 8, wobei das separate polare Prozesslösungsmittel dasselbe ist wie das Lösungsmittel.

10. Verfahren nach Anspruch 9, wobei der hydrophile Wirkstoff dem Lösungsmittel hinzugefügt wird.

11. Verfahren nach Anspruch 10, wobei der hydrophile Wirkstoff eine Nukleinsäure ist.

12. Verfahren nach Anspruch 11, wobei die Nukleinsäure ein Nukleinsäuresalz ist.

13. Verfahren nach Anspruch 11, wobei die Nukleinsäure mit einer neutralisierenden Base verbunden wird, bevor sie dem polaren Prozesslösungsmittel hinzugefügt wird.

**Revendications**

1. Nanoparticule comprenant :
un noyau hydrophile stabilisé par un polymère en peigne dont une partie hydrophile du polymère en peigne est orientée vers le centre de la nanoparticule.

2. Nanoparticule selon la revendication 1, dans laquelle la nanoparticule n'est pas réticulée, au moins une partie du noyau hydrophile est réticulée, ou une partie hydrophobe du polymère en peigne est réticulée.

3. Nanoparticule selon la revendication 1, dans laquelle la partie hydrophile du copolymère comprend un polymère non ionique.

4. Nanoparticule selon la revendication 3, dans laquelle le copolymère non ionique à partie hydrophile est un polysaccharide.

5. Nanoparticule selon la revendication 1, dans laquelle le noyau hydrophile comprend une petite molécule hydrosoluble, une protéine, un peptide, un acide nucléique, un polysaccharide ou une combinaison de ceux-ci.

6. Nanoparticule selon la revendication 5, dans laquelle l'acide nucléique est de l'ARN ou de l'ADN.

7. Procédé de fabrication d'une nanoparticule, comprenant les étapes consistant à :

former une première solution de traitement en dissolvant un polymère stabilisant présentant une partie hydrophile et une partie hydrophobe dans un flux de solvant polaire présentant un premier solvant de traitement ;
former une seconde solution de traitement en dissolvant un actif hydrophile dans un solvant polaire, qui peut être le premier solvant de traitement ou un solvant polaire différent ;

combiner l'actif hydrophile, le polymère stabilisant et le solvant avec un ou des flux hors traitement, où le ou les flux hors traitement contiennent un solvant supplémentaire qui est davantage non polaire que les solvants utilisés dans le premier ou le second flux de solvants de traitement ;

permettre à l'actif hydrophile de précipiter et de former un noyau hydrophile ; et

permettre à la partie hydrophile du polymère stabilisateur de précipiter sur le noyau hydrophile et à la partie hydrophobe de stabiliser le noyau.

dans lequel le polymère stabilisant est un polymère en peigne.

8. Procédé selon la revendication 7, dans lequel l'actif hydrophile est ajouté à un solvant de traitement polaire séparé et les deux flux de solvant de traitement et le flux de solvant hors traitement sont rapidement micromélangés dans un volume de mélange confiné dans des conditions de flux continu.

9. Procédé selon la revendication 8, dans lequel le solvant de traitement polaire séparé est identique au solvant.

10. Procédé selon la revendication 9, dans lequel l'actif hydrophile est ajouté au solvant.

11. Procédé selon la revendication 10, dans lequel l'actif hydrophile est un acide nucléique.

12. Procédé selon la revendication 11, dans lequel l'acide nucléique est un sel d'acide nucléique.

13. Procédé selon la revendication 11, dans lequel l'acide nucléique est combiné avec une base neutralisante avant d'être ajouté au solvant de traitement polaire.

**Process
Solvent Stream(s)**

Stabilizing
Block Polymer

Active (e.g. RNA):

Process Solvent
(e.g. DMSO)

**Non-Process
Solvent Stream(s)**

Non-Process Solvent
(e.g. DCM)

Rapid
Mixing

Inverse Nanoparticle

*FIG. 1*

**Process
Solvent Stream(s)**

Stabilizing
Comb Polymer

Active (e.g. RNA):

Process Solvent
(e.g. DMSO)

**Non-Process
Solvent Stream(s)**

Non-Process Solvent
(e.g. DCM)

Rapid
Mixing

Inverse Nanoparticle

*FIG. 2*

Polar Block(s):

Nonpolar Block(s) or Substituent(s):

Diblock Copolymer:

Triblock Copolymers:

Higher Order Block Copolymer:

*FIG. 3*

Linear Polar Backbone:

Nonpolar Side Chains(s) or Substituents:

Linear Comb Polymer(s):

*FIG. 4*

Non-Linear Polar Backbone:

Nonpolar Block(s) or Substituents:

Non-Linear Comb Polymer(s):

*FIG. 5*

Comb Polymer
Stabilized Inverse
Nanoparticle

Crosslinking
Agent

Comb Polymer
Stabilized Inverse
Nanoparticle, Core
Cross-Linked

Block Polymer
Stabilized Inverse
Nanoparticle

Block Polymer
Stabilized Inverse
Nanoparticle, Core
Cross-Linked

*FIG. 6*

Inverse Nanoparticle

Inverse Nanoparticle
With a Cross-Linked Shell

*FIG. 7*

*FIG. 8*

*FIG. 9*

*FIG. 10*

**FIG. 11**

**FIG. 12**

*FIG. 13*

*FIG. 14*

Collection Bath
+Ca$^{2+}$
+Nh$_3$

RNA, PAsp-PLA-PAsp,
DMSO/Water

*FIG. 15*

*FIG. 16*

FIG. 17

FIG. 18

*FIG. 19*

*FIG. 20*

FIG. 21

FIG. 22

**FIG. 23**

**FIG. 24**

*FIG. 25*

**REFERENCES CITED IN THE DESCRIPTION**

**Patent documents cited in the description**

- US 62895695
- WO 2017112828 A1
- WO 2015200054 A
- US 2019008788 A1
- US 2017209386 A1
- WO 2013188979 A1
- US 8137699 B
- US 81376992012 A, BK Johnson, RK Prud'homme
- US 13969449 B

**Non-patent literature cited in the description**

- **LIU, Y.** ; **CHENG, C** ; **PRUD'HOMME, R.K.** ; **FOX, R.O.** Mixing in a multi-inlet vortex mixer (MIVM) for flash nano-precipitation.. *Chemical Engineering Science*, 2008, vol. 63 (11), 2829-2842
- **JOHNSON, B.K.** ; **PRUD'HOMME, R.K.** *Chemical processing and micromixing in confined impinging jets. AIChE Journal*, 2003, vol. 49 (9), 2264-2282
- **D'ADDIO, S. M.** ; **PRUD'HOMME, R. K.** Controlling drug nanoparticle formation by rapid precipitation. *Advanced drug delivery reviews*, 2011, vol. 63 (6), 417-426
- **JOHNSON, B. K.** ; **PRUD'HOMME, R. K**. Process and apparatuses for preparing nanoparticle compositions with amphiphilic copolymers and their use. *Google Patents*, 2012
- **SAAD, W. S.** ; **PRUD'HOMME, R. K**. Principles of nanoparticle formation by flash nanoprecipitation.. *Nano Today*, 2016, vol. 11 (2), 212-227
- **PAGELS, R.F.** ; **PRUD'HOMME, R.K.** Polymeric nanoparticles and microparticles for the delivery of peptides, biologics, and soluble therapeutics.. *J Control Release*, 2015, vol. 219, 519-535
- NanoPrecipitation for Biologics Encapsulation: Nanoparticle Formation and Ionic Stabilization in Organic Solvents.. **PAGELS, R.F.** ; **PRUD'HOMME, R.K.** ; **INVERSE FLASH**. ACS Symposium Series. 2017, vol. 1271, 249-272
- Inverse Flash NanoPrecipitation for Biologics Encapsulation: Understanding Process Losses via an Extraction Protocol.. **MARKWALTER, C.E** ; **PRUD'HOMME, R.K.** ACS Symposium Series. 2017, vol. 1271, 275-296
- **SHIN, H. et al.** Recent advances in RNA therapeutics and RNA delivery systems based on nanoparticles.. *Adv. Therap.*, 2018, vol. 1, 180065
- **JOHNSON, B.K** ; **PRUD'HOMME, R.K.** Chemical processing and micromixing in confined impinging jets. *AIChE Journal*, 2003, vol. 49, 2264-2282
- **LIU, Y** ; **FOX, R.O.** CFD predictions for chemical processing in a confined impinging-jets reactor.. *AIChE Journal*, 2006, vol. 52, 731-744
- **LIU, Y.** ; **CHENG, C** ; **LIU, Y.** ; **PRUD'HOMME, R.K.** ; **FOX, R.O.** Mixing in a multi-inlet vortex mixer (MIVM) for flash nano-precipitation.. *Chemical Engineering Science*, 2008, vol. 63, 2829-2842
- **FAN, X. et al.** Recent Development of Unimolecular Micelles as Functional Materials and Applications.. *Polymer Chemistry*, 2016, vol. 7, 5898-5919
- **PAGELS, R.F.** ; **PRUD'HOMME, R.K.** Polymeric nanoparticles and microparticles for the delivery of peptides, biologics, and soluble therapeutics.. *J Control Release*, 2015, vol. 219, 519-535
- Inverse Flash NanoPrecipitation for Biologics Encapsulation: Nanoparticle Formation and Ionic Stabilization in Organic Solvents.. **PAGELS, R.F.** ; **PRUD'HOMME, R.K.** ACS Symposium Series. 2017, vol. 1271, 249-274
- NanoPrecipitation for Biologics Encapsulation: Understanding Process Losses via an Extraction Protocol.. **MARKWALTER, C.E.** ; **PRUD'HOMME, R.K.** ; **INVERSE FLASH**. ACS Symposium Series. 2017, vol. 1271, 275-296
- **BRESLOW, R.** ; **HUANG, D.-L.** Effects of metal ions, including Mg2+ and lanthanides, on the cleavage of ribonucleotides and RNA model compounds.. *Proc. Natl. Acad. Sci.*, 1991, vol. v88, 4080-4083
- Polymer Handbook. John Wiley & Sons, 1999
- Polymers in Controlled Drug Delivery Wright. 1987
- **ARSHADY**. *J. Controlled Release*, 1991, vol. 17, 1-22
- **PITT**. *Int. J. Phar.*, 1990, vol. 59, 173-196
- **HOLLAND et al.** *J. Controlled Release*, 1986, vol. 4, 155-0180
- **LAVASANIFAR, A.** Advanced Drug Delivery Reviews. 2002, vol. 54, 169-190

- **R. DAVIDSON**. Handbook of Water-Soluble Gums and Resins. McGraw-Hill, 1980

- Inverse Flash NanoPrecipitation for Biologics Encapsulation: Understanding Process Losses via an Extraction Protocol.. **MARKWALTER, C.E.** ; **PRUD'-HOMME, R.K**. ACS Symposium Series. 2017, vol. 1271, 275-296